# EUROPEAN PATENT APPLICATION

(11) **EP 4 656 197 A1**
(43) Date of publication of application: **03.12.2025**
(21) Application number: 24747417.4
(22) Date of filing: 22.01.2024
(51) Int. Cl.: A61K 35/28, A61P 17/14, A61K 8/98, A61Q 7/00, A61Q 5/00

(54) **COMPOSITION FOR TREATMENT OR PREVENTION OF HAIR LOSS COMPRISING STEM CELL-DERIVED EXOSOMES AND PREPARATION METHOD THEREFOR**

(30) Priority: 27.01.2023 KR 20230010608
(71) Applicant: Brexogen Inc., Seoul 05855 (KR)
(72) Inventor: KIM, Sue, Seoul 05855 (KR); OH, Hyun Geun, Seoul 05805 (KR)
(74) Representative: Plougmann Vingtoft a/s
(86) International application number: PCT/KR2024/001012
(87) International publication number: WO 2024/158180

(57) **Abstract**

The present invention relates to a composition for the treatment of hair loss, comprising stem cell-derived exosomes, and a preparation method therefor. The exosomes according to the present invention have an excellent growth and recovery promoting effect on human hair papilla cells, and thus can be used for various cosmetics and pharmaceutical compositions for the treatment of hair loss.

## Description

### Technical Field

The present disclosure relates to a composition for preventing or treating hair loss comprising stem cell-derived exosomes and a method for preparing the same and, more particularly, to a composition comprising exosomes isolated from mesenchymal stem cells or a culture thereof, which exhibit excellent effects in promoting the growth and recovery of human dermal papilla cells, thereby enabling the prevention or treatment of hair loss.

### Background Art

Extracellular vesicles are vesicles composed of spherical lipid bilayers with a size of about 30 to 1000 nm, including microvesicles, exosomes, and the like.

The lipid bilayer of an exosome has a phospholipid bilayer structure similar to that of the originating cell (donor) and is known to function in intercellular communication and immunological mediation.

Exosomes contain cell-specific components that reflect the biological function of the donor cell, and include phospholipids, mRNA, miRNA, and various soluble proteins, surface proteins, and transmembrane proteins.

Such exosomes are secreted from all types of animal cells including mast cells, lymphocytes, astrocytes, platelets, neurons, endothelial cells, and epithelial cells, and have been found in various body fluids such as blood, urine, mucus, saliva, bile, ascites, and cerebrospinal fluid. Exosomes can also pass through the blood-brain barrier (BBB). Due to the high selective permeability of exosomes, which allows them to permeate the membranes of epidermal and endothelial cells, exosomes are also utilized in the development of drug delivery systems (DDS) as nanocarriers for specific drugs.

Exosomes and microvesicles secreted by mesenchymal stem cells are known to be involved in cell-to-cell communication and exhibit the regenerative therapeutic efficacy associated with stem cells.

It has been reported that even without long-term survival after transplantation, stem cells can exert a trophic effect through paracrine factors secreted from the cells. These factors include low molecular weight substances such as growth factors, chemokines, and cytokines, which are secreted via extracellular vesicles like exosomes. Such exosomes are derived from stem cells and are thus used to identify the properties of stem cells and to evaluate their therapeutic efficacy. Furthermore, recent studies actively explore the therapeutic effects of exosomes secreted by mesenchymal stem cells without the use of the stem cells themselves, and both academia and industry expect that this approach may overcome the limitations of conventional stem cell therapy.

### Disclosure of Invention

### Technical Problem

Intensive and thorough research conducted by the present inventors developed a composition containing exosomes isolated from stem cells or a culture thereof and resulted in the finding that the composition exhibits excellent effects in promoting the growth and recovery of human dermal papilla cells, thereby enabling the prevention or treatment of hair loss.

Accordingly, an aspect of the present disclosure is to provide a composition containing stem cell-derived exosomes.

Another aspect of the present disclosure is to provide a pharmaceutical composition for preventing or treating hair loss, containing stem cell-derived exosomes.

Another aspect of the present disclosure is to provide a method for preventing or treating hair loss using stem cell-derived exosomes.

Another aspect of the present disclosure is to provide a use of a composition containing stem cell-derived exosomes for the prevention or treatment of hair loss.

Another aspect of the present disclosure is to provide a cosmetic composition containing stem cell-derived exosomes for preventing hair loss, strengthening hair roots, restoring hair follicles, or promoting hair growth.

Another aspect of the present disclosure is to provide a method for preventing hair loss, strengthening hair roots, restoring hair follicles, or promoting hair growth using stem cell-derived exosomes.

Another aspect of the present disclosure is to provide a use of a composition containing stem cell-derived exosomes for preventing hair loss, strengthening hair roots, restoring hair follicles, or promoting hair growth.

Another aspect of the present disclosure is to provide a method for preparing a composition containing stem cell-derived exosomes.

### Solution to Problem

The present disclosure relates to a composition for preventing or treating hair loss containing stem cell-derived exosomes and a method for preparing the same. The composition of the present disclosure enables the prevention or treatment of hair loss.

Hereinafter, a detailed description will be given of the present disclosure.

An aspect of the present disclosure provides a composition including stem cell-derived exosomes.

The term "exosome," as used herein, refers to cell-derived vesicles that are found in the bodily fluids of virtually all eukaryotic organisms. These vesicles are larger than LDL proteins but significantly smaller than red blood cells, typically having a diameter of approximately 30 to 100 nm. Exosomes are known to be released either when multivesicular bodies (MVBs) fuse with the cell membrane or directly from the cell membrane, and are recognized for their important and specialized roles in functions such as coagulation and intercellular signaling.

The term "stem cell," as used herein, refers to an undifferentiated cell capable of self-renewal and differentiation into two or more different types of cells.

In an embodiment of the present disclosure, the stem cell may be autologous or allogeneic, and may be derived from any type of animal, including humans and non-human mammals. It may be derived from adult tissues or from embryos. For example, the stem cell may be selected from the group consisting of adult stem cells, embryonic stem cells, induced pluripotent stem cells (iPSCs), mesenchymal stem cells derived from iPSCs, BxC stem cells, mesenchymal stem cells derived from iPSCs pretreated with hyaluronic acid (HA), and BxC-HA stem cells, but with no limitations thereto.

The term "adult stem cell," as used herein, refers to a cell extracted from umbilical cord blood, bone marrow, blood, or other sources from adults, which is in an undifferentiated state and capable of developing into tissue within the body when needed.

In an embodiment of the present disclosure, the adult stem cell may be selected from the group consisting of adult stem cells derived from humans, animals, or animal tissues; mesenchymal stromal cells derived from humans, animals, or animal tissues; and mesenchymal stromal cells derived from induced pluripotent stem cells of human or animal origin, but with no limitations thereto.

In the present disclosure, the human, animal, or animal tissue may be selected from the group consisting of umbilical cord, cord blood, bone marrow, adipose tissue, muscle, nerve, skin, amniotic membrane, and placenta, but is not limited thereto.

In the present disclosure, stem cells derived from various human or animal tissues may be selected from the group consisting of hematopoietic stem cells, mammary stem cells, intestinal stem cells, vascular endothelial stem cells, neural stem cells, olfactory stem cells, and testicular stem cells, but are not limited thereto.

The term "embryonic stem cell," as used herein, refers to a cell extracted during the developmental process of an embryo, specifically from the inner cell mass of a blastocyst-stage embryo prior to implantation in the uterus, and cultured in vitro.

Embryonic stem cells are cells that have the ability to self-renew and possess pluripotency or totipotency to differentiate into all types of tissues in an organism. In a broad sense, the term is also intended to encompass embryoid bodies (EBs) derived from embryonic stem cells.

In the present disclosure, the stem cell may include embryonic stem cells derived from any origin, including human, monkey, pig, horse, cow, sheep, dog, cat, mouse, and rabbit, but is not limited thereto.

The term "induced pluripotent stem cell (iPSC)," as used herein, refers to a cell reprogrammed from a differentiated cell through an artificial dedifferentiation process to acquire pluripotent differentiation capacity, and may be used interchangeably with the term "dedifferentiated stem cell."

The artificial dedifferentiation process may be performed by virus-mediated introduction using retroviruses, lentiviruses, or Sendai virus, or by non-viral methods such as the use of non-viral vectors, proteins, or cell extracts. It may also include dedifferentiation processes using stem cell extracts or small molecules.

Induced pluripotent stem cells exhibit properties nearly identical to those of embryonic stem cells. Specifically, they have similar morphology, gene and protein expression profiles, demonstrate pluripotency both in vitro and in vivo, form teratomas, can form chimera mice when injected into blastocysts, and are capable of germline transmission.

In an embodiment of the present disclosure, the stem cell may be a mesenchymal stem cell derived from an induced pluripotent stem cell.

The term "mesenchymal stem cell (MSC)," as used herein, refers to a stem cell derived from mesenchyme. MSCs can differentiate into one or more cell types selected from the group consisting of osteoblasts, chondrocytes, adipocytes, and myocytes. MSCs may be isolated from any type of adult tissue, for example, bone marrow, adipose tissue, umbilical cord, or peripheral blood. A MSC population may be defined by a characteristic phenotype. The population of mesenchymal stem cells differentiated from iPSCs may exhibit the same phenotypic characteristics as that of conventional MSCs. MSC populations may be understood as a group of stem cells expressing CD105, CD73, and CD90 markers at a level of 95% or higher, and expressing CD45, CD34, and SSEA-4 markers at a level of 2% or lower. In this context, for convenience, cells expressing CD45, CD34, or SSEA-4 at 2% or lower may be described as "not expressing" those markers.

In an embodiment of the present disclosure, the composition may include exosomes derived from induced pluripotent stem cell-derived mesenchymal stem cells.

In an embodiment of the present disclosure, the stem cell may be a BxC stem cell.

The term "BxC stem cell," as used herein, refers to a stem cell produced by isolating a population of induced pluripotent stem cells that do not express the SSEA-4 (stage-specific embryonic antigen 4) protein, followed by additional culturing. BxC stem cells are cells at a stage just before complete differentiation into mesenchymal stem cells from induced pluripotent stem cells, and they may acquire the full characteristics of mesenchymal stem cells through further culturing. Therefore, the phenotype of the BxC stem cell population may not exactly match that of mesenchymal stem cells, but it may be similar within a range of 96% to 99.9%. For example, an induced pluripotent stem cell population expresses CD90 protein at 0.3%, whereas mesenchymal stem cells express CD90 protein at 99.7%, and BxC stem cells express CD90 protein at a level of approximately 96.9%, which is about 98% of mesenchymal stem cells. Thus, BxC stem cells can be defined as stem cells that are differentiated to 96% to 99.9% of mesenchymal stem cells without fully differentiating them into mesenchymal stem cells after culturing induced pluripotent stem cells that do not express SSEA-4. BxC stem cells exhibit superior stemness and can secrete large amounts of functional proteins compared to mesenchymal stem cells derived from induced pluripotent stem cells. Specifically, the BxC stem cells of the present disclosure demonstrate more than a 10-fold increase in proliferation ability compared to mesenchymal stem cells derived from the same tissue after passing through 9 passages, and no decrease in proliferation ability is observed even after more than 12 passages. Additionally, BxC stem cells exhibit more than twice the expression of the proliferation marker Ki67 compared to general mesenchymal stem cells. Furthermore, BxC stem cells can express genes such as ANKRD1, CPE, NKAIN4, LCP1, CCDC3, MAMDC2, CLSTN2, SFTA1P, EPB41L3, PDE1C, EMILIN2, SULT1C4, TRIM58, DENND2A, CADM4, AIF1L, NTM, SHISA2, RASSF4, and ACKR3 at higher levels compared to mesenchymal stem cells derived from induced pluripotent stem cells. On the other hand, BxC stem cells can express genes such as DHRS3, BMPER, IFI6, PRSS12, RDH10, and KCNE4 at lower levels.

The term "stemness," as used herein, refers to the pluripotency and self-renewal ability of stem cells, which allows them to generate all types of cells and self-replicate indefinitely. This, for example, includes characteristics such as maintaining undifferentiated states while increasing stem cell proliferation, enhancing telomerase activity, increasing the expression of stemness-related signals, or increasing cell migration activity. One or more of these features may be exhibited.

In an embodiment of the present disclosure, the composition may comprise exosomes derived from BxC stem cells.

In an embodiment of the present disclosure, the stem cell may be a mesenchymal stem cell derived from an induced pluripotent stem cell pretreated with hyaluronic acid.

The term "pretreatment," as used herein, refers to a process of culturing mesenchymal stem cells in a medium to which a specific substance has been added. For example, pretreatment may refer to culturing mesenchymal stem cells, which have been fully differentiated from induced pluripotent stem cells, in a medium supplemented with hyaluronic acid.

In the present disclosure, hyaluronic acid may enhance the stemness and proliferative capacity of stem cells, and may increase the quantity of exosomes derived from stem cells as well as the protein and RNA content within the exosomes.

In an embodiment of the present disclosure, the composition may contain exosomes derived from mesenchymal stem cells that are derived from induced pluripotent stem cells and pretreated with hyaluronic acid.

In an embodiment of the present disclosure, the stem cell may be a BxC-HA stem cell.

The term "BxC-HA stem cell," as used herein, refers to a mesenchymal stem cell that has been fully differentiated from a BxC stem cell and subsequently cultured (pretreated) in a medium containing hyaluronic acid. For example, BxC-HA stem cells may be produced by further culturing BxC stem cells to achieve full differentiation into mesenchymal stem cells, and then culturing them in a medium containing 0.1 to 1000 µg/mL, for example 40 µg/mL, of hyaluronic acid for 12 to 48 hours. Compared to mesenchymal stem cells that have not undergone any pretreatment, BxC-HA stem cells exhibit approximately 360% higher proliferation rate, approximately five-fold increase in exosome production efficiency, and more than five-fold increase in the amount of exosome-derived proteins.

In an embodiment of the present disclosure, hyaluronic acid may be used for pretreatment at a concentration of 0.1 to 1000 µg/mL, 0.5 to 1000 µg/mL, 1 to 500 µg/mL, 1 to 200 µg/mL, 1 to 100 µg/mL, 1 to 80 µg/mL, 1 to 60 µg/mL, or 10 to 60 µg/mL, and for example, may be used at a concentration of 40 µg/mL, but is not limited thereto.

Another aspect of the present disclosure provides a pharmaceutical composition for the treatment, prevention, alleviation, or suppression of hair loss, including exosomes derived from mesenchymal stem cells originating from induced pluripotent stem cells as an active ingredient.

The term "hair loss," as used herein, refers to a condition in which hair is missing from areas where it is normally present or is abnormally reduced in amount, and more specifically, refers to a condition in which hair is missing or reduced on the scalp. Hair loss may occur or progress due to various internal or external factors. In the case of androgenetic alopecia, a typical form of hair loss, dihydrotestosterone (DHT) or testosterone, which are male hormones, bind to androgen receptors (AR) on dermal papilla cells that constitute hair follicles. This binding may regulate the expression of genes such as IGF1, EGF, FGF7, VEGF, IL-6, and TGF-β1, which are involved in hair growth in dermal papilla cells, thereby leading to hair loss.

The term "including (containing, comprising) as an active ingredient," as used herein, means that the composition includes a sufficient amount of exosomes isolated from stem cells or a culture thereof to achieve a specific effect, such as treatment or prevention of hair loss, or prevention of hair loss, strengthening of hair roots, restoration of hair follicles, or promotion of hair growth.

The pharmaceutical composition of the present disclosure may further include a pharmaceutically acceptable carrier.

The term "pharmaceutically acceptable," as used herein, refers to materials conventionally used in the pharmaceutical field that do not irritate the organism upon administration and do not impair the biological activity or properties of the administered compound.

In the present disclosure, any carrier commonly used in the relevant technical field may be employed. Nonlimiting examples of such carriers include saline, sterile water, Ringer's solution, buffered saline, albumin injection, lactose, dextrose, sucrose, sorbitol, mannitol, xylitol, erythritol, maltitol, maltodextrin, glycerol, ethanol, or combinations thereof.

In the present disclosure, the pharmaceutical composition may optionally include other pharmaceutically acceptable additives such as excipients, diluents, antioxidants, buffering agents, or bacteriostatic agents. Additionally, fillers, bulking agents, humectants, disintegrants, dispersants, surfactants, binders, or lubricants may be further added as needed.

In an embodiment of the present disclosure, the mesenchymal stem cells derived from induced pluripotent stem cells may be differentiated from precursor cells of induced pluripotent stem cell-derived mesenchymal stem cells that do not express the SSEA-4 (stage-specific embryonic antigen 4) protein.

In an embodiment of the present disclosure, the induced pluripotent stem cell may be human-derived.

In an embodiment of the present disclosure, the mesenchymal stem cell derived from induced pluripotent stem cells may be pretreated with a pretreatment substance.

In an embodiment of the present disclosure, the pretreatment substance may be hyaluronic acid.

Another aspect of the present disclosure provides a method for treating or preventing hair loss, the method including:
a step of administering or contacting exosomes isolated from mesenchymal stem cells derived from induced pluripotent stem cells to a subject.

The term "subject," as used herein, may refer to a mammal including humans, and may, for example, include a human, monkey, cow, horse, sheep, pig, cat, dog, mouse, rat, rabbit, or guinea pig, but is not limited thereto.

The term "administration," as used herein, refers to providing a predetermined substance to a subject by any appropriate method. The composition including exosomes of the present disclosure may be administered orally or non-orally through any conventional route, as long as it enables the composition to reach the target tissue. The composition may also be administered using any delivery device capable of delivering the active ingredient to target cells.

The term "contact," as used herein, refers to providing a predetermined substance to a subject through any appropriate non-invasive method. For example, the composition including exosomes of the present disclosure may be brought into direct or indirect contact with the epidermis or outer surface of the subject.

In an embodiment of the present disclosure, the exosomes may include BxC-HA stem cell-derived exosomes (BxC-HAe).

Another aspect of the present disclosure is the use of a pharmaceutical composition for the treatment or prevention of hair loss, including, as an active ingredient, exosomes isolated from mesenchymal stem cells derived from induced pluripotent stem cells.

Another aspect of the present disclosure provides a cosmetic composition for preventing hair loss, strengthening hair roots, restoring hair follicles, or promoting hair growth, including, as an active ingredient, exosomes isolated from mesenchymal stem cells derived from induced pluripotent stem cells.

The term "prevention of hair loss," as used herein, refers to delaying or completely suppressing the reduction of hair in specific areas by preventing the onset of hair loss in an individual.

The term "hair root," as used herein, refers to the portion of the hair located beneath the epidermis and within the dermis, not visible externally. The hair root includes the hair bulb at the lower part, the keratinized hair shaft produced from epithelial cells proliferated from the bulb, and the dermal papilla, which consists of dermal papilla cells that supply nutrients and regulate hair development and growth. Therefore, the term "strengthening of hair roots" refers to increasing the depth and thickness of the hair shaft within the root, increasing the size and viability of dermal papilla cells, and enhancing the expression of growth-promoting factors related to hair development.

The term "hair follicle," as used herein, refers to the structure located in the epidermis or subcutaneous tissue that surrounds and protects the hair root. Hair follicles are connected to capillaries and hair roots to provide nutrients to the roots, enabling continuous cell division and hair generation. Therefore, the term "restoration of hair follicles" refers to recovering the function of weakened or thinned hair follicles so that they can adequately protect and nourish the hair root.

The term "hair growth," as used herein, refers to the formation of hair on a body part, and more specifically, to the development and elongation of hair on the scalp. Accordingly, the term "promotion of hair growth" refers to inducing regrowth of missing or thinning hair on specific body parts such as the scalp, or enhancing the growth of hair with slowed development.

In the present disclosure, the cosmetic composition may have a formulation selected from the group consisting of solution, topical ointment, cream, foam, soap, liquid cleanser, bath additive, topical hair preparation, suspension, emulsion, paste, gel, lotion, powder, oil, patch, and spray, but is not limited thereto.

When the formulation of the present disclosure is an ointment, paste, cream, or gel, the carrier may include animal or vegetable oils, waxes, paraffin, starch, tragacanth, cellulose derivatives, polyethylene glycol, silicone, bentonite, silica, talc, zinc oxide, and the like. These may be used alone or in combination of two or more.

When the formulation is a powder or spray, carriers may include lactose, talc, silica, aluminum hydroxide, calcium silicate, polyamide powder, and the like. In particular, in the case of a spray, it may further include a propellant such as chlorofluorohydrocarbon, propane/butane, or dimethyl ether, but is not limited thereto. These may be used alone or in combination of two or more.

When the formulation is a solution or emulsion, the carrier may include a solvent, solubilizer, or emulsifier. For example, water, ethanol, isopropanol, ethyl carbonate, ethyl acetate, benzyl alcohol, benzyl benzoate, propylene glycol, 1,3-butylene glycol, and oils such as cottonseed oil, peanut oil, corn germ oil, olive oil, castor oil, sesame oil, glycerol fatty acid esters, polyethylene glycol, or fatty acid esters of sorbitan may be used, but are not limited thereto. These may be used alone or in combination of two or more.

When the formulation is a suspension, the carrier may include a liquid diluent such as water, ethanol, or propylene glycol; a suspending agent such as ethoxylated isostearyl alcohol, polyoxyethylene sorbitol ester, or polyoxyethylene sorbitan ester; or other agents such as microcrystalline cellulose, aluminum metahydroxide, bentonite, agar, or tragacanth. These may be used alone or in combination of two or more.

When the formulation is a topical hair preparation, the cosmetic composition may specifically be in the form of a hair tonic, hair conditioner, hair essence, hair lotion, hair nutrient lotion, hair shampoo, hair rinse, hair treatment, hair cream, hair nutrient cream, hair moisture cream, hair massage cream, hair wax, hair aerosol, hair pack, hair nutrient pack, hair soap, hair cleansing foam, hair oil, hair drying agent, hair preservative, hair dye, hair waving agent, hair bleach, hair gel, hair glaze, hair dressing, hair lacquer, hair moisturizer, hair mousse, or hair spray.

In an embodiment of the present disclosure, the mesenchymal stem cell derived from induced pluripotent stem cells may be differentiated from precursor cells of mesenchymal stem cells that do not express the SSEA-4 (stage-specific embryonic antigen 4) protein.

In an embodiment of the present disclosure, the induced pluripotent stem cell may be human-derived.

In an embodiment of the present disclosure, the mesenchymal stem cell derived from induced pluripotent stem cells may be pretreated with a pretreatment substance.

In an embodiment of the present disclosure, the pretreatment substance may be hyaluronic acid.

Another aspect of the present disclosure provides a method for preventing hair loss, strengthening hair roots, restoring hair follicles, or promoting hair growth, the method comprising:
a step of administering or contacting exosomes isolated from induced pluripotent stem cell-derived mesenchymal stem cells derived from s to a subject.

Another aspect of the present disclosure is a use of a cosmetic composition including, as an active ingredient, exosomes isolated from induced pluripotent stem cell-derived mesenchymal stem cells for preventing hair loss, strengthening hair roots, restoring hair follicles, or promoting hair growth.

Another aspect of the present disclosure provides a method for preparing the composition, the method comprising:
a step of isolating exosomes from stem cells or a culture thereof.

In an embodiment of the present disclosure, the isolation step may involve isolating exosomes from induced pluripotent stem cell-derived mesenchymal stem cells or from a culture thereof.

In the isolation step, the culture medium of the stem cells may first be centrifuged at 200-400×g for 5 to 20 minutes to remove remaining cells and cell debris, followed by collecting the supernatant and performing high-speed centrifugation at 9,000-12,000×g for 60 to 80 minutes. The resulting supernatant may then be centrifuged again at 90,000-120,000×g for 80 to 100 minutes, and the exosomes remaining in the pellet after removal of the supernatant may be collected.

In an embodiment of the present disclosure, the stem cell may be autologous or allogeneic, may be derived from any animal including humans and non-human mammals, and may be adult-derived or embryonic-derived. For example, the stem cell may be an adult stem cell, embryonic stem cell, induced pluripotent stem cell, induced pluripotent stem cell-derived mesenchymal stem cell, BxC stem cell, or BxC-HA stem cell, but is not limited thereto.

In an embodiment of the present disclosure, the method may further include a pretreatment step of pretreating the induced pluripotent stem cell-derived mesenchymal stem cells with hyaluronic acid.

In an embodiment of the present disclosure, the method may further include a selection culture step of isolating and culturing SSEA-4-negative [SSEA-4(-)] cells that do not express the SSEA-4 protein among cultured induced pluripotent stem cells, and differentiating them into BxC stem cells.

In an embodiment of the present disclosure, the method may further include a production step of culturing stem cells in a cell culture medium.

The production step according to the present disclosure refers to a process of inducing the secretion or production of exosomes from stem cells. In the present disclosure, the cell culture medium may include any medium conventionally used for culturing stem cells in the art. Examples include commercially available or synthetically prepared media such as DMEM (Dulbecco's Modified Eagle's Medium), MEM (Minimal Essential Medium), BME (Basal Medium Eagle), RPMI 1640, DMEM/F-10 (Dulbecco's Modified Eagle's Medium: Nutrient Mixture F-10), DMEM/F-12 (Dulbecco's Modified Eagle's Medium: Nutrient Mixture F-12), α-MEM (α-Minimal Essential Medium), G-MEM (Glasgow's Minimal Essential Medium), IMDM (Iscove's Modified Dulbecco's Medium), KnockOut DMEM, and E8 (Essential 8 Medium), but is not limited thereto.

In an embodiment of the present disclosure, the cell culture medium may further include components such as carbon sources, nitrogen sources, trace elements, amino acids, and antibiotics.

In an embodiment of the present disclosure, the production step may include an additional culturing step in which the stem cells are cultured using exosome-depleted fetal bovine serum (FBS). Unlike regular FBS, which contains a large amount of bovine serum-derived exosomes, exosome-depleted FBS is free of such exosomes and thus prevents contamination of the medium with exosomes derived from bovine serum rather than those secreted from the stem cells.

Another aspect of the present disclosure provides a method for preparing a pharmaceutical composition for the treatment of hair loss, the method including:
a first culturing step of culturing induced pluripotent stem cells in a medium;
a selectively culturing step of isolating SSEA-4(-) cells, which do not express the SSEA-4 protein, from the cultured induced pluripotent stem cells and culturing same to differentiate into BxC stem cells;
a second culturing step of culturing the BxC stem cells to differentiate into mesenchymal stem cells;
a pretreatment step of pretreating the mesenchymal stem cells with hyaluronic acid;
a production step of culturing the pretreated mesenchymal stem cells to produce exosomes; and
a collection step of collecting the culture supernatant from the mesenchymal stem cells or a culture thereof.

In the present disclosure, the method may further include an isolation step of isolating exosomes from the culture supernatant.

In an embodiment of the present disclosure, the first culturing step may include culturing the induced pluripotent stem cells in a medium containing FBS and bFGF (basic Fibroblast Growth Factor) for 1 to 10 days.

In an embodiment of the present disclosure, the selectively culturing step may include isolating SSEA-4(-) cells that do not express the SSEA-4 protein among the induced pluripotent stem cells and culturing same in a medium containing FBS and bFGF for 1 to 10 days to differentiate same into BxC stem cells.

In an embodiment of the present disclosure, the pretreatment step may include culturing the mesenchymal stem cells in a medium containing hyaluronic acid at a concentration of 0.1 to 1000 µg/mL, 0.5 to 1000 µg/mL, 1 to 500 µg/mL, 1 to 200 µg/mL, 1 to 100 µg/mL, 1 to 80 µg/mL, 1 to 60 µg/mL, or 10 to 60 µg/mL, and, for example, at 40 µg/mL.

In an embodiment of the present disclosure, the production step may include an additional culturing step in which the mesenchymal stem cells are cultured in exosome-depleted FBS.

In an embodiment of the present disclosure, the collection step may further include a step of additionally culturing the pretreated mesenchymal stem cells, collecting the culture medium, removing cells and cell debris, and obtaining the culture supernatant.

In an embodiment of the present disclosure, the isolation step may be carried out by collecting the supernatant and performing high-speed centrifugation at 9,000-12,000×g for 60 to 80 minutes, followed by collecting the supernatant again and centrifuging same at 90,000-120,000×g for 80 to 100 minutes, and then removing the supernatant to obtain exosomes remaining in the pellet.

### Advantageous Effects of Invention

The present disclosure relates to a composition for treating hair loss comprising stem cell-derived exosomes and a preparation method therefor. The exosomes according to the present disclosure exhibit excellent effects in promoting the growth and recovery of human dermal papilla cells and can be utilized as pharmaceutical compositions for treating hair loss and in various cosmetic formulations.

### Brief Description of Drawings

FIG. 1 is a graph showing the results of a comparative experiment on the recovery of cell viability in human dermal papilla cells (treated with DHT) following treatment with exosomes (BxC-HAe) derived from hyaluronic acid-pretreated induced pluripotent stem cell-derived mesenchymal stem cells (BxC-HA) according to an embodiment of the present disclosure.
FIG. 2 is a graph showing the results of a comparative experiment on the recovery of cell viability in human dermal papilla cells (treated with testosterone) following BxC-HAe treatment.
FIG. 3 is a set of images comparing the degree of wound healing immediately and 24 hours after treatment with BxC-HAe in a monolayer of wounded human dermal papilla cells (treated with DHT), in comparison to a control.
FIG. 4 is a graph showing the results of a comparative experiment on wound recovery in human dermal papilla cells (treated with DHT) following BxC-HAe treatment.
FIG. 5 is a graph showing the changes in IGF1 gene expression in human dermal papilla cells (treated with DHT) after treatment with BxC-HAe.
FIG. 6 is a graph showing the changes in EGF gene expression in human dermal papilla cells (treated with DHT) after treatment with BxC-HAe.
FIG. 7 is a graph showing the changes in IGF1 gene expression in human dermal papilla cells (treated with testosterone) after treatment with BxC-HAe.
FIG. 8 is a graph showing the changes in FGF7 gene expression in human dermal papilla cells (treated with testosterone) after treatment with BxC-HAe.
FIG. 9 is a graph showing the changes in VEGF gene expression in human dermal papilla cells (treated with testosterone) after treatment with BxC-HAe.
FIG. 10 is a graph showing the changes in TGF-β1 gene expression in human dermal papilla cells (treated with DHT) after treatment with BxC-HAe.
FIG. 11 is a graph showing the changes in IL-6 gene expression in human dermal papilla cells (treated with DHT) after treatment with BxC-HAe.
FIG. 12 is a graph showing the changes in TGF-β1 gene expression in human dermal papilla cells (treated with testosterone) after treatment with BxC-HAe.
FIG. 13 is a graph showing the changes in IL-6 gene expression in human dermal papilla cells (treated with testosterone) after treatment with BxC-HAe.
FIG. 14 is a graph showing the changes in AR gene expression in human dermal papilla cells (treated with DHT) after treatment with BxC-HAe.
FIG. 15 is a graph showing the changes in AR gene expression in human dermal papilla cells (treated with testosterone) after treatment with BxC-HAe.
FIG. 16 is a graph showing changes in body weight in mice (treated with testosterone) following BxC-HAe treatment.
FIGS. 17 and 18 are graphs showing changes in hair growth area in mice (treated with testosterone) following BxC-HAe treatment.
FIG. 19 is an image comparing the hair growth area in mice (treated with testosterone) following BxC-HAe treatment versus a control group.
FIG. 20 is an H&E-stained image of skin tissue from mice (treated with testosterone) following BxC-HAe treatment, compared with the control group.
FIG. 21 is a graph showing changes in the ratio of anagen-phase hair follicles in skin tissue of mice (treated with testosterone) after BxC-HAe treatment.
FIG. 22 is an immunostained image of skin tissue from mice (treated with testosterone) following BxC-HAe treatment, using antibodies.
FIG. 23 is a graph showing the fluorescence intensity of AR in immunostained skin tissue from mice (treated with testosterone) following BxC-HAe treatment.
FIG. 24 is a graph showing the fluorescence intensity of β-catenin in immunostained skin tissue from mice (treated with testosterone) following BxC-HAe treatment.
FIG. 25 is a set of images comparing wound healing immediately and 24 hours after treating a monolayer of wounded human dermal papilla cells (treated with testosterone) with conditioned medium (CM) from BxC-HA cells.
FIG. 26 is a graph showing the wound recovery rate of human dermal papilla cells (treated with testosterone) following CM treatment.
FIG. 27 is a graph showing changes in IGF1 gene expression in human dermal papilla cells (treated with testosterone) following CM treatment.
FIG. 28 is a graph showing changes in EGF gene expression in human dermal papilla cells (treated with testosterone) following CM treatment.
FIG. 29 is a graph showing changes in TGF-β1 gene expression in human dermal papilla cells (treated with testosterone) following CM treatment.
FIG. 30 is a graph showing changes in IL-6 gene expression in human dermal papilla cells (treated with testosterone) following CM treatment.

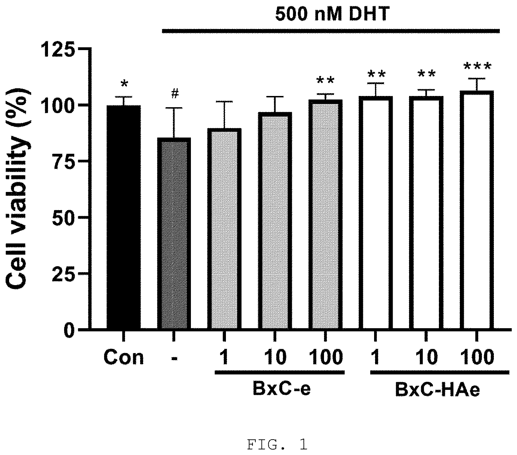

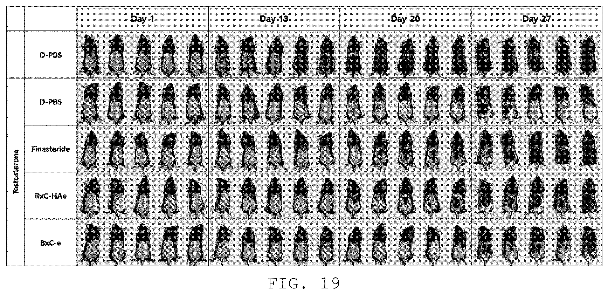

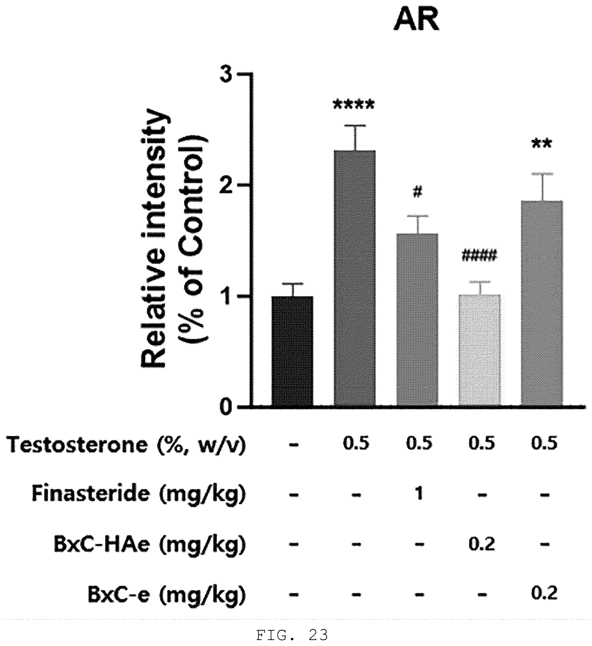

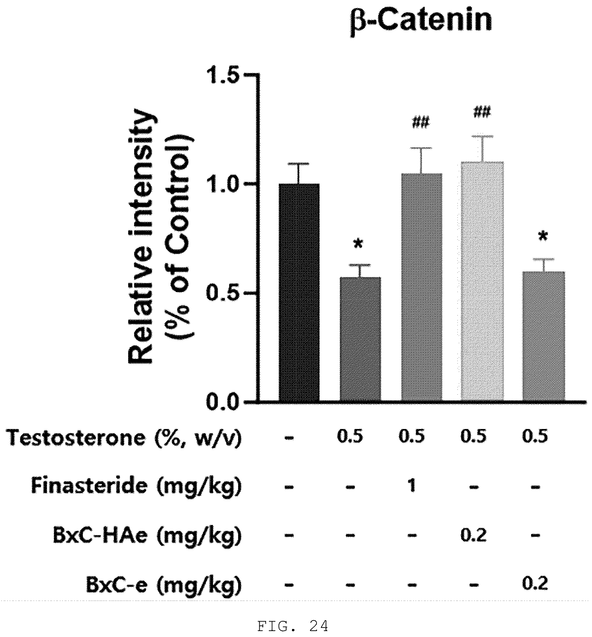

In FIGS. 1 to 30, statistical analysis of the control and experimental groups was conducted using one-way ANOVA. In each figure, "* or #" indicates p < 0.05, "** or ##" indicates p < 0.01, "*** or ###" indicates p < 0.001, and "**** or ####" indicates p < 0.0001.

### Best Mode for Carrying out the Invention

A pharmaceutical composition for the treatment, prevention, alleviation, or suppression of hair loss, including, as an active ingredient, exosomes isolated from induced pluripotent stem cell (iPSC)-derived mesenchymal stem cells.

### Mode for Carrying out the Invention

In the following description, ~ A better understanding of the present disclosure may be obtained through the following examples, which are set forth to illustrate, but are not to be construed to limit the scope of the present disclosure.

### EXAMPLE 1: Culture of Induced Pluripotent Stem Cell-Derived Mesenchymal Stem Cells

Induced pluripotent stem cells (iPSCs) were cultured for 7 days in DMEM supplemented with 10% FBS (Fetal Bovine Serum) and 10 ng/mL bFGF (basic Fibroblast Growth Factor). Subsequently, SSEA-4(-) cells, which do not express the SSEA-4 (stage-specific embryonic antigen 4) protein on the cell surface, were isolated by FACS analysis from the cultured iPSCs to obtain precursor cells of iPSC-derived mesenchymal stem cells. The isolated SSEA-4(-) cells were then subcultured for an additional 7 days in DMEM medium containing 10% FBS and 10 ng/mL bFGF to produce BxC stem cells.

Thereafter, the BxC stem cells were further cultured in a medium containing High Glucose DMEM (Gibco, USA), 10% FBS (HyClone, USA), and 1% MEM Non-Essential Amino Acids Solution (100X, Gibco, USA) to induce full differentiation into induced pluripotent stem cell-derived mesenchymal stem cells.

### EXAMPLE 2: Isolation of Hyaluronic Acid-Pretreated Exosomes (BxC-HAe)

The mesenchymal stem cells derived from iPSCs obtained in Example 1 were cultured for 24 hours in High Glucose DMEM medium supplemented with 10% FBS, 1% MEM Non-Essential Amino Acids Solution, and 40 µg/mL hyaluronic acid to prepare hyaluronic acid-pretreated iPSC-derived mesenchymal stem cells (BxC-HA stem cells).

After culturing, the BxC-HA stem cells were washed and further cultured for 72 hours in medium supplemented with 10% exosome-depleted FBS.

After 72 hours of culturing, the medium containing the pretreatment substance was collected and centrifuged at 300×g for 10 minutes to remove residual cells and debris. The supernatant was then collected and filtered through a 0.22 µm filter, followed by centrifugation using a high-speed centrifuge at 10,000×g for 70 minutes at 4°C. The resulting supernatant was collected again and centrifuged using an ultracentrifuge at 100,000×g for 90 minutes at 4°C. After removing the supernatant, the remaining pellet containing exosomes was resuspended in PBS to isolate hyaluronic acid-pretreated exosomes (hereinafter referred to as BxC-HAe), which were used in the following experiments.

### EXAMPLE 3: Recovery of Reduced Cell Viability in Human Dermal Papilla Cells (DHT Treatment)

To confirm whether the composition of the present disclosure restores the reduced cell viability of human dermal papilla cells, the cell proliferation rate was compared 24 hours after treatment with BxC-HAe, which was isolated in Example 2.

Specifically, 1,000 human dermal papilla cells were seeded per well in a 96-well plate and incubated in basal medium at 37°C with 5% CO₂ for 24 hours. The composition of the basal medium is shown in Table 1 below.

**TABLE 1**

| Product Name | Specification |
|---|---|
| Follicle Dermal Papilla Cell-Basal Medium(PromoCell C-26500) | - |
| Follicle Dermal Papilla Cell Growth Medium SupplementPack (PromoCell C-39620) | FCS 0.04 mℓ/mℓ (Fetal Calf Serum) |
| | BPE-26 0.004 mℓ/mℓ (Bovine |
| | Pituitary Extract) |
| | HbFGF 1 ng/mℓ (Human basic Fibroblast Growth Factor) |
| | Insulin 5 *µ*g/mℓ |
| Antibiotic-Antimycotic (100X, liquid) (GenDEPOT CA002-010) | - |

Next, to establish control and experimental groups, the dihydrotestosterone (DHT)-untreated group received 0.05% (v/v) DMSO in the basal medium. The control group was treated with 500 nM DHT in the basal medium. Experimental Group 1 was treated with 500 nM DHT and 1 to 100 µg/mL of Test Substance 1 (BxC-e). Experimental Group 2 was treated with 500 nM DHT and 1 to 100 µg/mL of Test Substance 2 (BxC-HAe). After treatment, all samples were incubated for 24 hours.

After incubation, total cell viability was measured using the Cell Count Kit-8 (CCK-8) assay (Enzo, New York, NY, USA). Specifically, after 24 hours of incubation, CCK-8 solution was added to each well, followed by incubation at 37°C for 2 hours. Optical density (OD) was then measured at 450 nm using a microplate reader. The results are shown in FIG. 1 and Table 2 below. The measured values from each well (n = 5 per group) were averaged, and the average value of the control group was set to 100% for relative comparison.

**TABLE 2**

| | DHT untreated | 500 nM DHT treated | | | | | | |
|---|---|---|---|---|---|---|---|---|
| | | Contro l | 1^{st} Test Group (BxC-e treated, *µ*g/mℓ) | | | 2^{nd} Test Group (BxC-HAe treated, *µ*g/mℓ) | | |
| | | | 1 | 10 | 100 | 1 | 10 | 100 |
| Cell Viability(%) | 100 | 85.4 | 89. 8 | 96. 9 | 102. 4 | 104. 0 | 104. 0 | 106. 5 |

From the results, it was observed that DHT treatment reduced cell viability by approximately 15% compared to the untreated group. Treatment with 100 µg/mL of BxC-e resulted in a 2.4% increase in cell viability relative to the untreated group. In contrast, treatment with BxC-HAe resulted in a 4.0% increase in cell viability at 1 µg/mL or 10 µg/mL, and a 6.5% increase at 100 µg/mL, confirming that BxC-HAe significantly improved cell viability compared to the control group.

### EXAMPLE 4: Recovery of Reduced Cell Viability in Human Dermal Papilla Cells (Testosterone Treatment)

To confirm whether the composition of the present disclosure restores the reduced cell viability of human dermal papilla cells, the cell proliferation rate was compared 24 hours after treatment with BxC-HAe, which was isolated in Example 2.

Specifically, 1,000 human dermal papilla cells were seeded per well in a 96-well plate and incubated in basal medium at 37°C under 5% CO₂ for 24 hours. The composition of the basal medium is shown in Table 3 below.

**TABLE 3**

| Product name | Specification |
|---|---|
| Follicle Dermal Papilla-Cell Basal Medium(PromoCell C-26500) | - |
| Follicle Dermal Papilla Cell Growth Medium SupplementPack (PromoCell C-39620) | FCS 0.04 mℓ/mℓ (Fetal Calf Serum) |
| | BPE-26 0.004 mℓ/mℓ (Bovine Pituitary Extract) |
| | HbFGF 1 ng/mℓ (Human basic Fibroblast Growth Factor) |
| | Insulin 5 *µ*g/mℓ |
| Antibiotic-Antimycotic | - |
| (100X, liquid) (GenDEPOT CA002-010) | |

Next, for the control and experimental groups, the testosterone untreated group was treated with 0.05% (v/v) DMSO in the basal medium. The control group was treated with 50 µM testosterone in the basal medium. Experimental Group 1 was treated with 50 µM testosterone and 10 to 50 µg/mL of Test Substance 1 (BxC-e). Experimental Group 2 was treated with 50 µM testosterone and 10 to 50 µg/mL of Test Substance 2 (BxC-HAe). After treatment, each group was incubated for 24 hours.

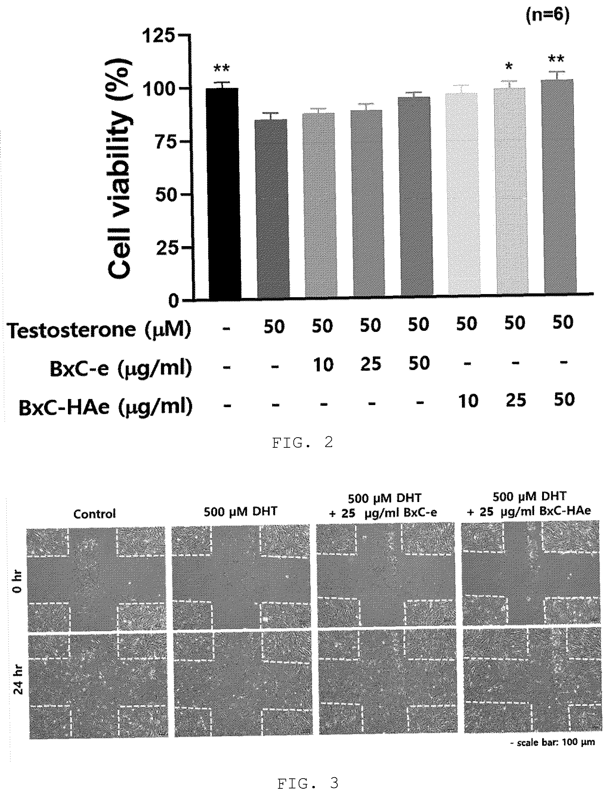

After incubation, total cell viability was measured using the Cell Count Kit-8 (CCK-8) assay (Enzo, New York, NY, USA). Specifically, after 48 hours of incubation, CCK-8 solution was added to each well, followed by a 2-hour incubation at 37°C. Optical density (OD) was measured at 450 nm using a microplate reader. The results are shown in FIG. 2 and Table 4 below. The measured values for each well (n = 6 per group) were averaged, and relative values were expressed based on the control group average set to 100%.

**TABLE 4**

| | DHT-untreated | 500 nM DHT-treated | | | | | | |
|---|---|---|---|---|---|---|---|---|
| | | Control | Test group (BxC-e treated, *µ*g/mℓ) | | | 2^{nd} Test group (BxC-HAe treated, *µ*g/mℓ) | | |
| | | | 10 | 25 | 50 | 10 | 25 | 50 |
| Cell viability (%) | 100 | 84.78 | 87.22 | 88.42 | 94.16 | 95.55 | 97.42 | 101.5 |

The results showed that testosterone treatment decreased cell viability by approximately 15% compared to the untreated group. Treatment with 50 µg/mL of BxC-e led to approximately 6% decrease in cell viability relative to the untreated group. Meanwhile, treatment with BxC-HAe at 10 µg/mL or 25 µg/mL resulted in approximately 4% or 2% decrease, respectively, in cell viability compared to the untreated group. However, treatment with 50 µg/mL BxC-HAe led to a 1.5% increase in cell viability compared to the untreated group, demonstrating a significant improvement in cell viability relative to the control group.

### EXAMPLE 5: Recovery of Reduced Wound Healing Rate in Human Dermal Papilla Cells

To determine whether the composition of the present disclosure restores the reduced wound healing rate in human dermal papilla cells, the wound healing rate was compared 24 hours after treatment with BxC-HAe, which was isolated in Example 2.

Specifically, 25,000 human dermal papilla cells were seeded per well in a 12-well plate and incubated in basal medium at 37°C under 5% CO₂ for 24 hours. The composition of the basal medium was the same as in Table 1 above. After a cell monolayer had formed through incubation, a cross-shaped wound was created using a 1 mL pipette tip, and the wells were washed three times with 1× D-PBS to remove cell debris.

For the control and experimental groups, the DHT-untreated group was treated with 0.05% (v/v) DMSO in the basal medium. The control group was treated with 500 nM DHT. Experimental Group 1 was treated with 500 nM DHT and 25 µg/mL of Test Substance 1 (BxC-e). Experimental Group 2 was treated with 500 nM DHT and 25 µg/mL of Test Substance 2 (BxC-HAe). After treatment, the cells were incubated for an additional 24 hours.

To observe the healing of the cell layer in each medium, changes of the cell layer were monitored at the time of drug treatment and after 24 hours using the Dixi eXcope system. The images for each group are shown in FIG. 3.

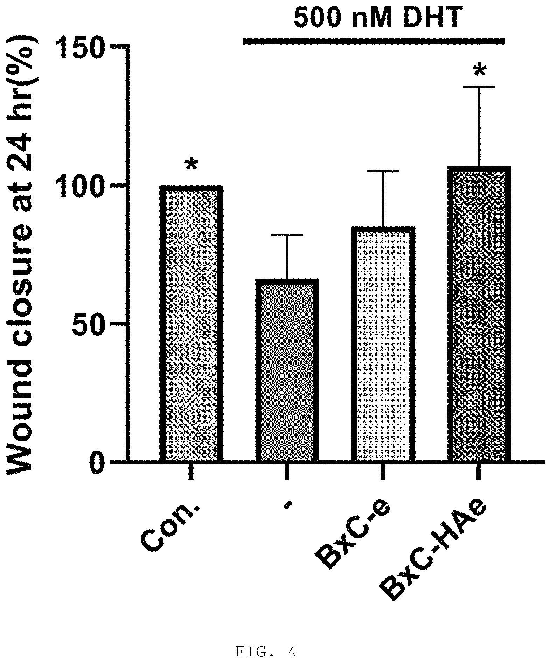

The captured images were quantitatively analyzed using ImageJ software. Each control and experimental group was tested in five replicates, and the average values were calculated and shown in FIG. 4 and Table 5. The average value of each group was expressed as a relative value based on 100% for the DHT-untreated group.

**TABLE 5**

| | DHT-untreated | 500 nM DHT-treated | | |
|---|---|---|---|---|
| | | Control | 1^{st} Test group (BxC-e treated) | 2^{nd} Test group (BxC-HAe treated) |
| Wound healing recovery after 24 hrs (%) | 100 | 66.16 | 85.21 | 107.0 |

According to the analysis, wound healing rate decreased by approximately 34% in the DHT-treated group compared to the DHT-untreated group. Treatment with the first test substance, BxC-e, led to approximately a 15% decrease in wound healing rate compared to the DHT-untreated group. In contrast, treatment with the second test substance, BxC-HAe, resulted in a 7% increase in wound healing rate compared to the DHT-untreated group.

While the increase in wound healing rate in Experimental Group 1 was not statistically significant compared to the control group, Experimental Group 2 showed a statistically significant increase in wound healing rate compared to the control group. These results confirm that BxC-HAe significantly promotes wound healing in human dermal papilla cells.

### EXAMPLE 6: Promotion of Growth Factor Expression in Human Dermal Papilla Cells (DHT Treatment)

To determine whether the composition of the present disclosure promotes the expression of IGF1 (Insulin-like Growth Factor 1) and EGF (Epidermal Growth Factor), which are known to be critical growth factors for hair growth, the expression levels of IGF1 and EGF genes were compared 24 hours after treatment with BxC-HAe (isolated in Example 2) in human dermal papilla cells.

Specifically, 25,000 human dermal papilla cells were seeded per well in a 12-well plate and incubated in basal medium at 37°C under 5% CO₂ for 24 hours. The composition of the basal medium was the same as described in Table 1 above.

For the control and experimental groups, the DHT-untreated group received 0.05% (v/v) DMSO in the basal medium. The control group was treated with 500 nM DHT. Experimental Group 1 was treated with 500 nM DHT and 10 µg/mL of Test Substance 1 (BxC-e). Experimental Group 2 was treated with 500 nM DHT and 10 µg/mL of Test Substance 2 (BxC-HAe). All groups were incubated for 24 hours after treatment.

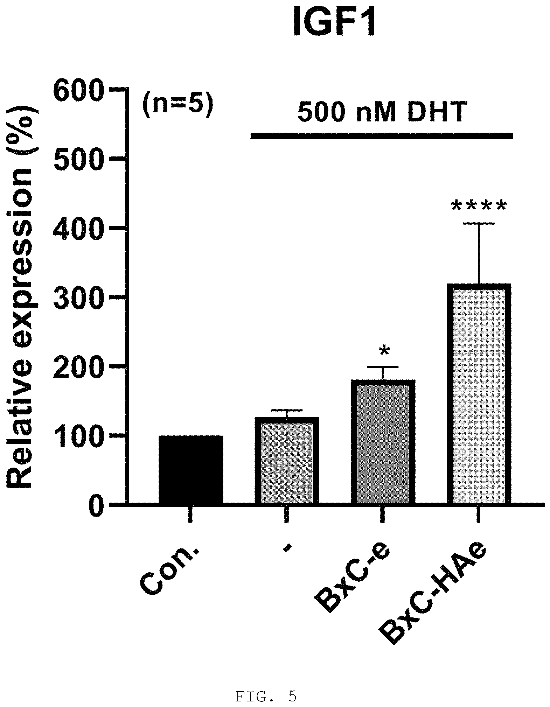

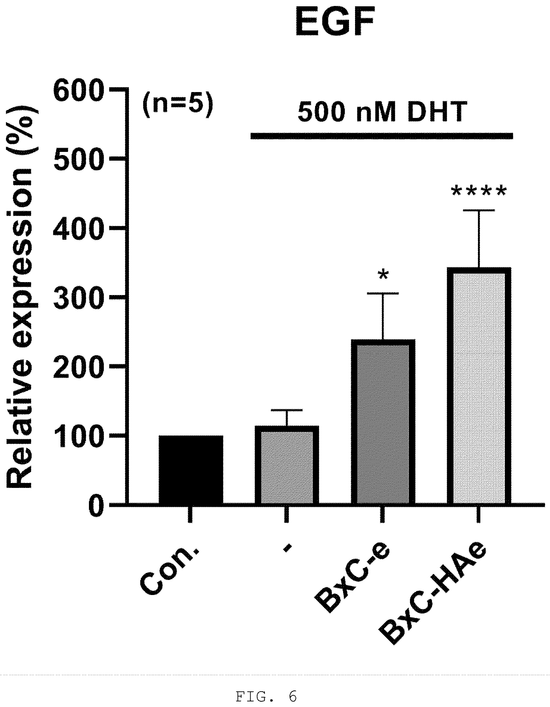

After incubation, cells from each medium were collected, and total RNA was extracted using TRIzol^{™} (Invitrogen). The mRNA expression levels of IGF1 and EGF were measured using Real-Time PCR (Applied Biosystems) based on equal amounts of total RNA from each sample. The expression levels were normalized to GAPDH using the 2^{-ΔΔCt} method, and relative quantification was performed by setting the expression levels in the DHT-untreated group to 100%. The quantification results for IGF1 and EGF expression are shown in FIG. 5 and Table 6, and in FIG. 6 and Table 7, respectively.

**TABLE 6**

| | DHT-untreated | 500 nM DHT-treated | | |
|---|---|---|---|---|
| | | Control | 1^{st} Test group (BxC-e treated) | 2^{nd} Test group (BxC-HAe treated) |
| Relative expression of IGF1 gene (%) | 100 | 126.8 | 181.4 | 319.8 |

**TABLE 7**

| | DHT-untreated | 500 nM DHT-treated | | |
|---|---|---|---|---|
| | | Control | 1^{st} Test group (BxC-e treated) | 2^{nd} Test group (BxC-HAe treated) |
| Relative expression of EGF gene (%) | 100 | 114.0 | 239.5 | 343.2 |

The quantification results showed that, upon DHT treatment, IGF1 gene expression increased to 126.8% and EGF gene expression to 114.0% relative to the DHT-untreated group. Upon treatment with the first test substance (BxC-e), IGF1 expression increased to 181.4% and EGF expression to 239.5% compared to the DHT-untreated group.

Treatment with the second test substance (BxC-HAe) resulted in a 319.8% increase in IGF1 expression and a 343.2% increase in EGF expression relative to the DHT-untreated group. These increases were found to be statistically significant compared to the control group. Therefore, it was confirmed that BxC-HAe can promote hair growth by enhancing the expression of IGF1 and EGF in human dermal papilla cells, and thus may be effective for the treatment or prevention of hair loss.

### EXAMPLE 7: Promotion of Growth Factor Expression in Human Dermal Papilla Cells (Testosterone Treatment)

To determine whether the composition of the present disclosure promotes the expression of IGF1 (Insulin-like Growth Factor 1), FGF7 (Fibroblast Growth Factor 7), and VEGF (Vascular Endothelial Growth Factor), which are known as key growth factors for hair growth, human dermal papilla cells were treated with BxC-HAe (isolated in Example 2), and the expression levels of IGF1, FGF7, and VEGF genes were compared 24 hours later.

Specifically, 25,000 human dermal papilla cells were seeded per well in a 12-well plate and incubated in basal medium at 37°C under 5% CO₂ for 24 hours. The composition of the basal medium was the same as in Table 1.

For the control and experimental groups, the testosterone-untreated group was treated with 0.05% (v/v) DMSO in the basal medium. The control group was treated with 50 µM testosterone. Experimental Group 1 was treated with 50 µM testosterone and 100 nM of Test Substance 1 (Finasteride). Experimental Group 2 was treated with 50 µM testosterone and 25 µg/mL of Test Substance 2 (BxC-e). Experimental Group 3 was treated with 50 µM testosterone and 25 µg/mL of Test Substance 3 (BxC-HAe). All groups were incubated for 24 hours after treatment.

Here, Finasteride refers to a compound commonly used in the treatment of androgenetic alopecia that inhibits androgens, and the same meaning is used hereinafter.

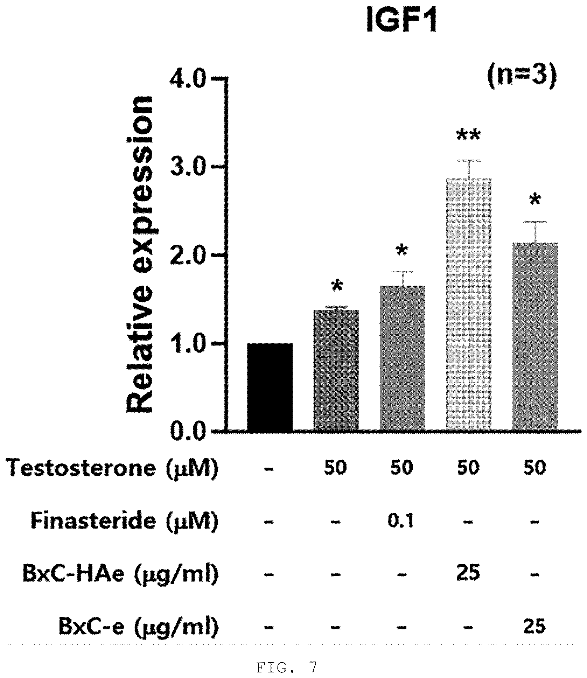

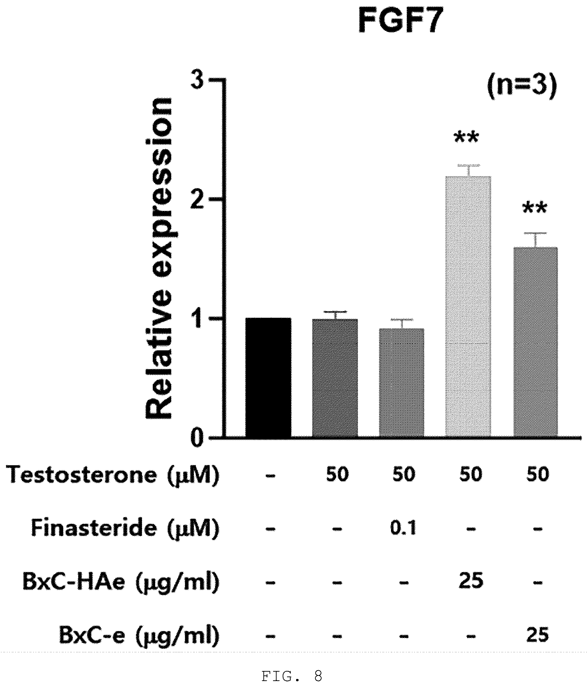

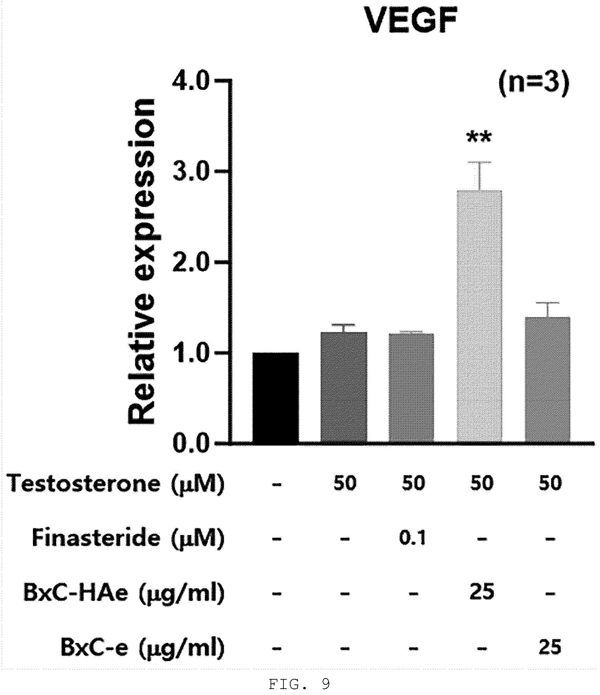

After incubation, cells were collected from each medium, and total RNA was extracted using TRIzol^{™} (Invitrogen). Using Real-Time PCR (Applied Biosystems), the mRNA expression levels of the IGF1, FGF7, and VEGF genes were measured based on equal amounts of total RNA from each sample. Expression values were normalized to GAPDH using the 2^{-ΔΔCt} method, and relative expression levels were calculated by setting the value for the testosterone-untreated group to 100%. The quantification results for IGF1, FGF7, and VEGF expression were presented in FIG. 7 and Table 8, FIG. 8 and Table 9, and FIG. 9 and Table 10, respectively.

**TABLE 8**

| | Testosterone -untreated | 50 uM Testosterone-treated | | | |
|---|---|---|---|---|---|
| | | Contro l | 1^{st} Test group (Finasteride -treated) | 2^{nd} Test group (BxC-e treated ) | 3^{rd} Test group (BxC-HAe treated ) |
| Relative expressio n of IGF1 gene (%) | 100 | 137 | 162 | 214 | 287 |

**TABLE 9**

| | Testosterone -untreated | 50 uM Testosterone-treated | | | |
|---|---|---|---|---|---|
| | | Contro l | 1^{st} Test group (Finasteride -treated) | 2^{nd} Test group (BxC-e treated ) | 3^{rd} Test group (BxC-HAe-treated ) |
| Relative expressio n of FGF7 gene (%) | 100 | 99 | 91 | 160 | 219 |

**TABLE 10**

| | Testosterone -untreated | 50 uM Testosterone-treated | | | |
|---|---|---|---|---|---|
| | | Contro l | 1^{st} Test group (Finasterid e treated) | 2^{nd} Test group (BxC-e treated ) | 3^{rd} Test group (BxC -HAe treated) |
| Relative expressio n of VEGF gene (%) | 100 | 122 | 122 | 140 | 279 |

The results showed that, compared to the testosterone-untreated group, testosterone treatment increased IGF1 expression to 137% and VEGF expression to 122%, while FGF7 expression decreased to 99%. Upon treatment with Test Substance 1 (Finasteride), IGF1 and VEGF expression increased to 162% and 122%, respectively, while FGF7 expression decreased to 91%. In contrast, treatment with Test Substance 2 (BxC-e) increased IGF1, FGF7, and VEGF expression to 214%, 160%, and 140%, respectively, compared to the testosterone-untreated group.

Furthermore, treatment with Test Substance 3 (BxC-HAe) increased IGF1 expression to 287%, FGF7 expression to 219%, and VEGF expression to 279% compared to the untreated group. These increases were found to be statistically significant compared to the control group. These findings confirm that BxC-HAe significantly promotes the expression of IGF1, FGF7, and VEGF in human dermal papilla cells, and thus may promote hair growth and provide therapeutic or preventive effects against hair loss.

### EXAMPLE 8: Inhibition of Hair Follicle Growth Suppressor Gene Expression in Human Dermal Papilla Cells (DHT Treatment)

To determine whether the composition of the present disclosure suppresses the expression of TGF-β1 (Transforming Growth Factor-beta 1) and IL-6 (Interleukin-6)-known to shorten the anagen phase and induce the catagen and telogen phases of hair follicles-human dermal papilla cells were treated with BxC-HAe (isolated in Example 2), and the expression levels of TGF-β1 and IL-6 were compared after 24 hours.

Specifically, 25,000 human dermal papilla cells were seeded per well in a 12-well plate and incubated in basal medium at 37°C under 5% CO₂ for 24 hours. The composition of the basal medium was the same as in Table 1.

For the control and experimental groups, the DHT-untreated group received 0.05% (v/v) DMSO in the basal medium. The control group was treated with 500 nM DHT. Experimental Group 1 was treated with 500 nM DHT and 10 µg/mL of Test Substance 1 (BxC-e). Experimental Group 2 was treated with 500 nM DHT and 10 µg/mL of Test Substance 2 (BxC-HAe). After treatment, each group was incubated for 24 hours.

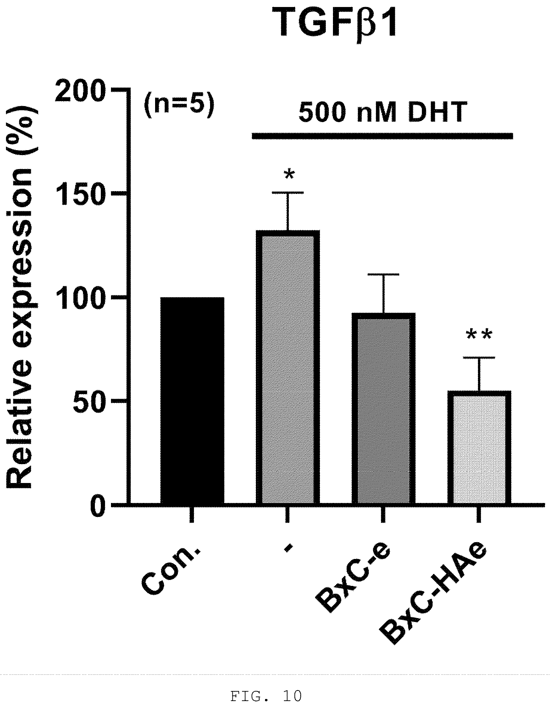

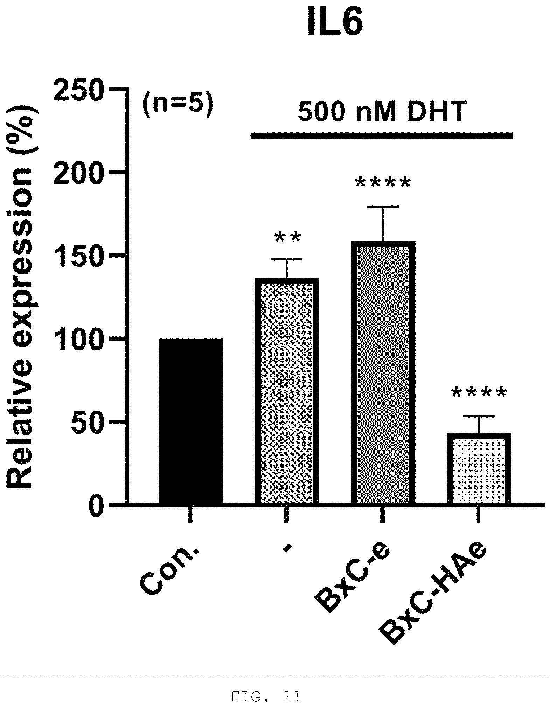

After incubation, cells were collected from each medium, and total RNA was extracted using TRIzol^{™} (Invitrogen). The mRNA expression levels of the TGF-β1 and IL-6 genes were measured using Real-Time PCR (Applied Biosystems) based on equal amounts of RNA extracted from each group. Expression values were normalized to GAPDH using the 2^{-ΔΔCt} method, and relative quantification was calculated by setting the DHT-untreated group to 100%. The results for TGF-β1 and IL-6 gene expression are shown in FIG. 10 and Table 11, and in FIG. 11 and Table 12, respectively.

**TABLE 11**

| | DHT-untreated | 500 nM DHT-treated | | |
|---|---|---|---|---|
| | | Control | 1^{st} Test group (BxC-e treated) | 2^{nd} Test group (BxC-HAe treated) |
| Relative expression of TGF-β1 gene (%) | 100 | 132.5 | 92.6 | 55.3 |

**TABLE 12**

| | DHT-untreated | 500 nM DHT-treated | | |
|---|---|---|---|---|
| | | Control | 1^{st} Test group (BxC-e treated) | 2^{nd} Test group (BxC-HAe treated) |
| Relative expression of IL-6 gene (%) | 100 | 136.4 | 158.9 | 43.7 |

According to the quantification results: for TGF-β1, DHT treatment increased gene expression by 132.5% compared to the DHT-untreated group. Treatment with BxC-e decreased TGF-β1 expression by 92.6% compared to the DHT-untreated group; and for IL-6, DHT treatment increased expression by 136.4% compared to the untreated group, while treatment with BxC-e further increased IL-6 expression by 158.9% compared to the untreated group.

In contrast, treatment with BxC-HAe (Test Substance 2) reduced TGF-β1 expression by 55.3% and IL-6 expression by 43.7% compared to the DHT-untreated group. These reductions were statistically significant compared to the control group. Therefore, it was confirmed that BxC-HAe effectively suppresses the expression of both TGF-β1 and IL-6, which inhibit hair follicle growth, thereby helping to maintain the anagen (growth) phase of the hair follicle.

### EXAMPLE 9: Inhibition of Hair Follicle Growth Suppressor Gene Expression in Human Dermal Papilla Cells (Testosterone Treatment)

To determine whether the composition of the present disclosure suppresses the expression of TGF-β1 (Transforming Growth Factor-beta 1) and IL-6 (Interleukin-6), which are known to shorten the anagen phase and induce the catagen and telogen phases, human dermal papilla cells were treated with BxC-HAe (isolated in Example 2), and the gene expression levels were compared 24 hours later.

Specifically, 25,000 human dermal papilla cells were seeded per well in a 12-well plate and incubated in basal medium at 37°C under 5% CO₂ for 24 hours. The composition of the basal medium was the same as described in Table 1.

For the control and experimental groups, the testosterone-untreated group received 0.05% (v/v) DMSO in the basal medium. The control group was treated with 50 µM testosterone. Experimental Group 1 was treated with 50 µM testosterone and 100 nM of Test Substance 1 (Finasteride). Experimental Group 2 was treated with 50 µM testosterone and 25 µg/mL of Test Substance 2 (BxC-e). Experimental Group 3 was treated with 50 µM testosterone and 25 µg/mL of Test Substance 3 (BxC-HAe). All groups were incubated for 24 hours after treatment.

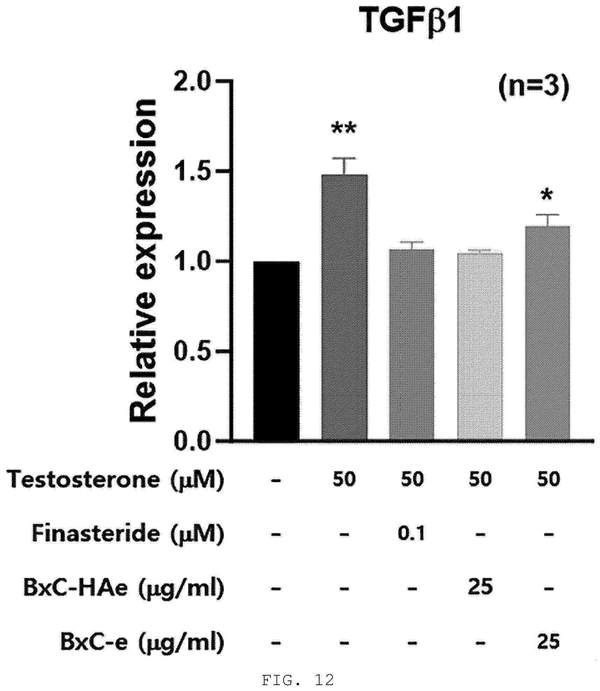

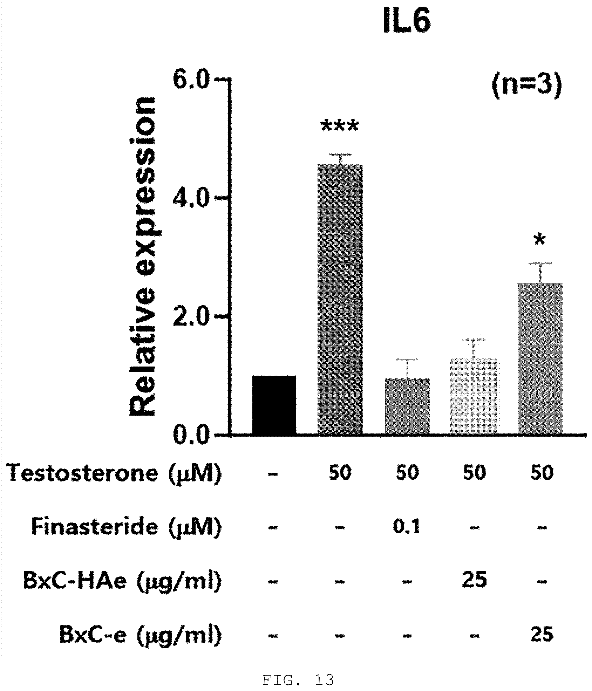

After incubation, cells from each group were harvested, and total RNA was extracted using TRIzol^{™} (Invitrogen). The mRNA expression levels of TGF-β1 and IL-6 were measured by Real-Time PCR (Applied Biosystems) using equal amounts of RNA from each sample. Expression levels were normalized to GAPDH using the 2^{-ΔΔCt} method, and relative quantification was calculated by setting the expression levels of the testosterone-untreated group to 100%. The quantification results for TGF-β1 and IL-6 were presented in FIG. 12 and Table 13, and FIG. 13 and Table 14, respectively.

**TABLE 13**

| | Testosterone -untreated | 50 uM Testosterone -treated | | | |
|---|---|---|---|---|---|
| | | Contro l | 1^{st} Test group (Finasterid e treated) | 2^{nd} Test group (BxC-e treated ) | 3^{rd} Test group (BxC-HAe treated ) |
| Relative expressio n of TGF-β1 gene (%) | 100 | 148 | 107 | 119 | 105 |

**TABLE 14**

| | Testosterone -untreated | 50 uM Testosterone -treated | | | |
|---|---|---|---|---|---|
| | | Contro l | 1^{st} Test group (Finasterid e treated) | 2^{nd} Test group (BxC-e treated ) | 3^{rd} Test group (BxC-HAe treated ) |
| Relative expressio n of IL-6 gene (%) | 100 | 456 | 95 | 257 | 130 |

The quantitative results were as follows: For TGF-β1, testosterone treatment increased expression to 148% compared to the testosterone untreated group. When applied, the first test drug finasteride treatment increased expression to 107%, the second test drug BxC-e to 119%, and the third test drug BxC-HAe to 105%, all relative to the untreated group.

For IL-6, testosterone, when applied to human dermal papilla cells, treatment resulted in a dramatic increase in expression to 456% compared to the testosterone untreated group. The first test drug finasteride reduced expression to 95%, the second test drug BxC-e increased expression to 257%, while the third test drug BxC-HAe led to a more moderate increase to 130% compared to the untreated group.

That is, treatment with BxC-HAe significantly reduced the expression levels of both TGF-β1 and IL-6 compared to the control group, and the differences were statistically significant. Therefore, BxC-HAe was confirmed to suppress the expression of TGF-β1 and IL-6-key inhibitors of hair follicle growth-and thereby help sustain the hair follicle's anagen phase.

### EXAMPLE 10: Androgen Receptor (AR) Expression Inhibition in Human Dermal Papilla Cells (DHT Treatment)

To determine whether the composition of the present disclosure suppresses the expression of androgen receptor (AR), which is the receptor for DHT, in human dermal papilla cells, the expression levels of AR were compared 24 hours after treatment with BxC-HAe, which was isolated in Example 2.

Specifically, 25,000 human dermal papilla cells were seeded per well in a 12-well plate and incubated in basal medium at 37°C under 5% CO₂ for 24 hours. The composition of the basal medium was the same as described in Table 1.

For the control and experimental groups, the DHT-untreated group received 0.05% (v/v) DMSO in the basal medium. The control group was treated with 500 nM DHT. Experimental Group 1 was treated with 500 nM DHT and 10 µg/mL of Test Substance 1 (BxC-e). Experimental Group 2 was treated with 500 nM DHT and 10 µg/mL of Test Substance 2 (BxC-HAe). After treatment, all groups were incubated for 24 hours.

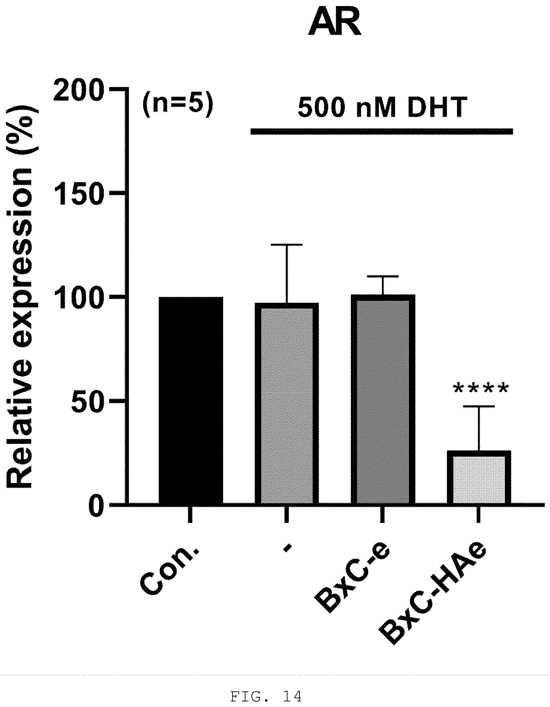

After incubation, total RNA was extracted from each group using TRIzol^{™} (Invitrogen), and the mRNA expression levels of the AR gene were measured using Real-Time PCR (Applied Biosystems) based on equal amounts of RNA. Expression levels were normalized to GAPDH using the 2^{-ΔΔCt} method, and relative quantification was calculated by setting the value of the DHT-untreated group to 100%. The results of AR gene expression quantification are shown in FIG. 14 and Table 15.

**TABLE 15**

| | DHT - untreated | 500 nM DHT -treated | | |
|---|---|---|---|---|
| | | Control | 1^{st} Test group (BxC-e treated) | 2^{nd} Test group (BxC-HAe treated) |
| Relative expression of AR gene (%) | 100 | 97.5 | 101.3 | 26.3 |

According to the results, treatment with DHT reduced AR gene expression by 97.5% compared to the DHT-untreated group. In contrast, treatment with BxC-e increased AR gene expression to 101.3% of the level observed in the DHT-untreated group.

Treatment with BxC-HAe reduced AR gene expression by 26.3% compared to the DHT-untreated group. This reduction was statistically significant compared to the control group, indicating that BxC-HAe markedly suppresses AR gene expression. Therefore, it was confirmed that BxC-HAe reduces the sensitivity of human dermal papilla cells to DHT, suggesting that it may be effective in the treatment or prevention of androgenetic alopecia caused by DHT.

### EXAMPLE 11: Inhibition of Androgen Receptor Expression in Human Dermal Papilla Cells (Testosterone Treatment)

To determine whether the composition of the present disclosure suppresses the expression of the androgen receptor (AR), which is the receptor for testosterone, human dermal papilla cells were treated with BxC-HAe isolated in Example 2, and AR gene expression was compared after 24 hours.

Specifically, 25,000 human dermal papilla cells were seeded per well in a 12-well plate and incubated in basal medium at 37°C under 5% CO₂ for 24 hours. The composition of the basal medium was the same as described in Table 1.

For the control and experimental groups: the testosterone-untreated group was treated with 0.05% (v/v) DMSO; the control group was treated with 50 µM testosterone; Experimental Group 1 was treated with 50 µM testosterone and 100 nM of Test Substance 1 (Finasteride); Experimental Group 2 was treated with 50 µM testosterone and 25 µg/mL of Test Substance 2 (BxC-e); Experimental Group 3 was treated with 50 µM testosterone and 25 µg/mL of Test Substance 3 (BxC-HAe). Each group was incubated for 24 hours following treatment.

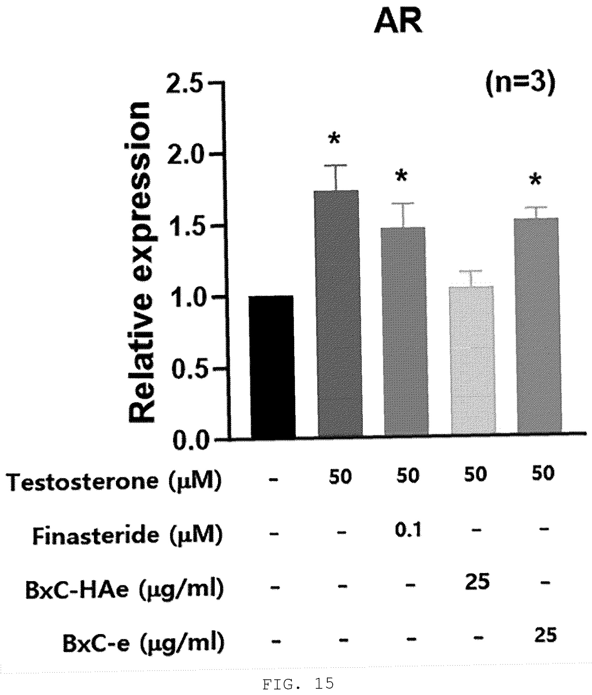

After incubation, cells were collected from each well, and total RNA was extracted using TRIzol^{™} (Invitrogen). The mRNA expression level of the AR gene was measured by Real-Time PCR (Applied Biosystems) using equal amounts of total RNA. The results were normalized to GAPDH using the 2^{-ΔΔCt} method, and relative expression was calculated by setting the value of the testosterone-untreated group to 100%. The quantification results for AR expression are shown in FIG. 15 and Table 16.

**TABLE 16**

| | Testosterone -untreated | 50 uM Testosterone-treated | | | |
|---|---|---|---|---|---|
| | | Contr ol | 1^{st} Test group (Finasteri de treated) | 2^{nd} Test group (BxC-e treated) | 3^{rd} Test group (BxC-HAe treated) |
| Relative expressio n of AR gene (%) | 100 | 173 | 146 | 151 | 104 |

The quantification results showed that treatment with testosterone increased AR gene expression to 173% compared to the testosterone-untreated group. Treatment with Finasteride increased AR expression to 146%, and treatment with BxC-e increased AR expression to 151% relative to the testosterone-untreated group.

In contrast, treatment with BxC-HAe resulted in an AR expression level of 104% compared to the testosterone-untreated group. This was a statistically significant reduction compared to the control group, confirming that BxC-HAe can reduce the sensitivity of human dermal papilla cells to testosterone and may be effective in the treatment or prevention of androgenetic alopecia induced by testosterone.

### EXAMPLE 12: Hair Regrowth in Testosterone-Treated Animal Model

To evaluate whether the composition of the present disclosure promotes hair regrowth, BxC-HAe isolated in Example 2 was administered to a testosterone-treated animal model, and changes in body weight and photographic observations were compared.

Specifically, six-week-old C57BL/6 mice were acclimated for two weeks under conditions of 25°C and 50% humidity with ad libitum access to food and tap water. Eight-week-old mice were then used for the experiment.

On Day 0, initial depilation was performed using a hair clipper, followed by the application of Niklin (Il-dong Pharmaceutical) for 90 seconds for secondary depilation. The area was then rinsed three times with lukewarm water.

In the testosterone-treated group, starting on Day 1, 100 µL of 0.5% (w/w) testosterone dissolved in 50% ethanol was topically applied daily to the depilated dorsal region of each mouse.

For control and experimental groups, beginning on Day 1 and continuing every other day: the control group received subcutaneous injections of 100 µL D-PBS; Experimental Group 1 received 100 µL D-PBS containing 1 mg/kg of Test Substance 1 (Finasteride); Experimental Group 2 received 100 µL D-PBS containing 0.2 mg/kg of Test Substance 2 (BxC-e); and Experimental Group 3 received 100 µL D-PBS containing 0.2 mg/kg of Test Substance 3 (BxC-HAe) .

Starting from the point when hair regrowth began in the testosterone-untreated group, body weight was measured twice weekly, and photographs were taken once weekly.

Specifically, hair regrowth area and depilated area were measured using ImageJ software, and the ratio of hair regrowth area to depilated area was calculated and analyzed statistically.

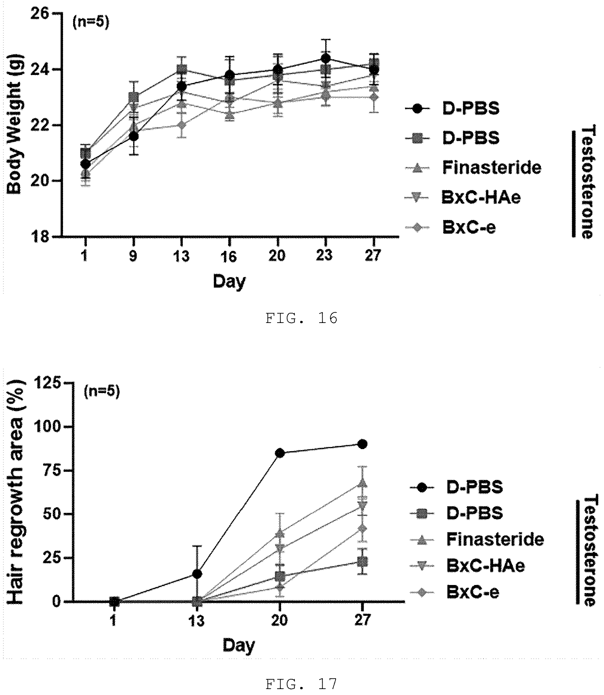

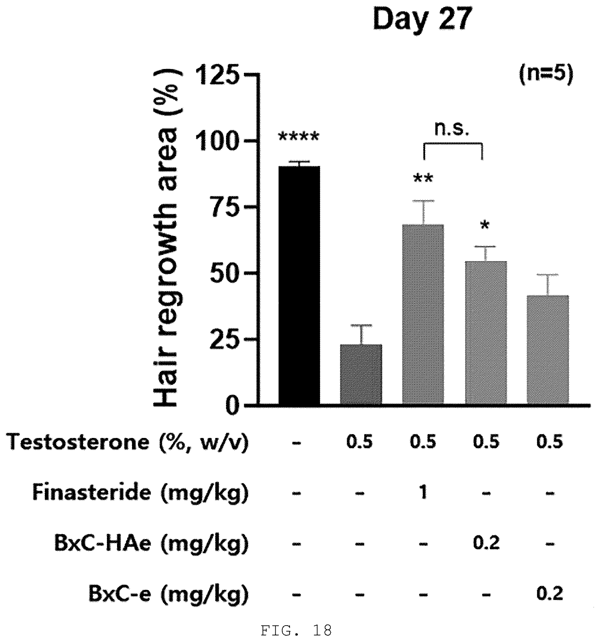

The experiment was conducted until Day 27, the point at which hair regrowth was completed in the testosterone-untreated group. The results for body weight are shown in FIG. 16, and hair regrowth area results are shown in FIGS. 17 and 18, and Table 17.

**TABLE 17**

| | Testosteron e-untreated | Testosterone-treated | | | |
|---|---|---|---|---|---|
| | | Contro l | 1^{st} Test group 1 (Finasteride treated) | 2^{nd} Test group (BxC-e treated) | 3^{rd} Test group 3 (BxC-HAe treated) |
| Hair growth area (%) | 100 | 23.0 | 68.1 | 41.8 | 54.7 |

According to the quantification results: on Day 27, hair regrowth area in the testosterone-treated group was reduced to 23.0% compared to the testosterone-untreated group; with Finasteride (Test Substance 1) treatment, hair regrowth area recovered to 68.1% of that in the untreated group; and with BxC-e (Test Substance 2), the regrowth area was 41.8%.

Notably, Test Substance 3 (BxC-HAe) treatment resulted in 54.7% hair regrowth area relative to the testosterone-untreated group, and this was a statistically significant improvement compared to the control group. These results confirm that BxC-HAe exhibits a significantly superior effect on hair regrowth even in the presence of testosterone, indicating its potential for the treatment or prevention of androgenetic alopecia induced by testosterone.

### EXAMPLE 13: Inhibition of Hair Follicle Reduction in Testosterone-Treated Animal Model

To determine whether the composition of the present disclosure inhibits the reduction of hair follicles, the ratio of anagen-phase hair follicles was measured in a testosterone-treated animal model following administration of BxC-HAe isolated in Example 2.

Specifically, dorsal skin tissues from each mouse subjected to the 27-day experiment described in Example 8 were collected, embedded in paraffin blocks, and sectioned at a thickness of 5 µm. The tissue sections were then stained using H&E.

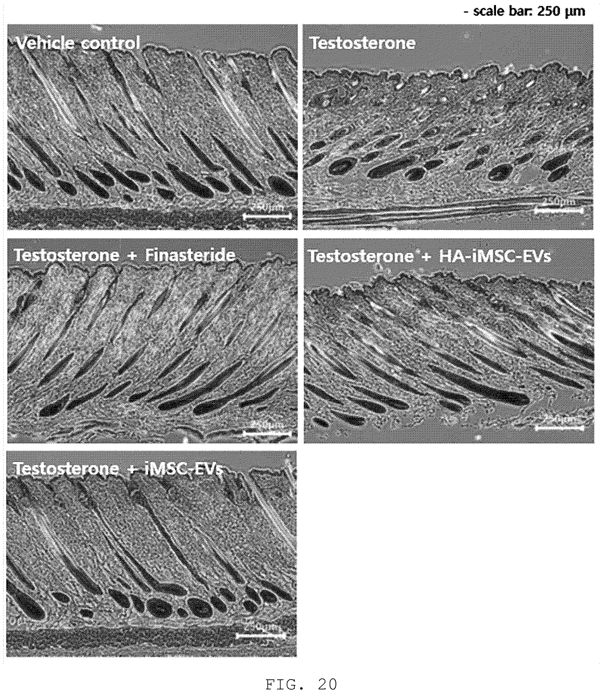

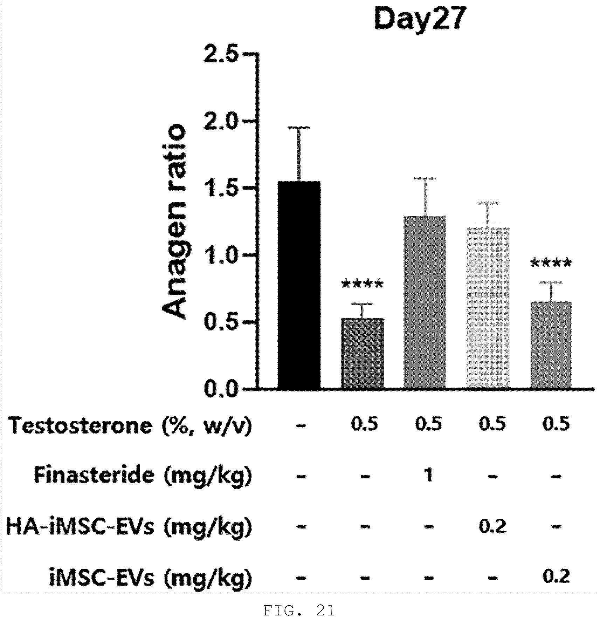

Subsequently, for each group, randomly selected hair follicles from the dorsal skin of five mice were classified by morphology into anagen (growth phase), catagen (regression phase), and telogen (resting phase). The ratio of anagen hair follicles was calculated using the formula: Anagen / (Catagen + Telogen). The results were presented in FIGS. 20 and 21, and Table 18.

**TABLE 18**

| | Testosterone -untreated | Testosterone-treated | | | |
|---|---|---|---|---|---|
| | | Contro l | 1^{st} Test group 1 (Finasterid e treated) | 2^{nd} Test group (BxC-e treated ) | 3^{rd} Test group (BxC-HAe treated ) |
| Percentag e of hair follicles in growth phase (%) | 1.55 | 0.53 | 1.29 | 0.65 | 1.20 |
| Total No. of hair follicles calculate d (n) | 92 | 117 | 118 | 95 | 103 |

According to the quantification results: in the testosterone-untreated group, the anagen follicle ratio was 1.55%; in the testosterone-treated group, this ratio decreased to 0.53%; with Test Substance 1 (Finasteride) treatment, the ratio recovered to 1.29%; with Test Substance 2 (BxC-e) treatment, the ratio was 0.65%; and with Test Substance 3 (BxC-HAe) treatment, the ratio increased to 1.20%, significantly higher than that in the testosterone-treated control group.

Notably, even under testosterone treatment, BxC-HAe demonstrated a remarkably superior inhibitory effect on follicle reduction, indicating that BxC-HAe may be effective in the treatment or prevention of androgenetic alopecia caused by testosterone.

### EXAMPLE 14: Cells Marked by Hair Loss and Hair Regrowth Factors in Testosterone-Treated Animals

To determine whether the composition of the present disclosure modulates the expression of hair loss and regrowth-related factors, a testosterone-treated animal model was administered BxC-HAe isolated in Example 2, and the fluorescence intensity of cells stained with antibodies against androgen receptor (AR) and β-catenin was measured and compared.

Specifically, dorsal skin tissues from each mouse subjected to the 27-day experiment (described in Example 8) were harvested, embedded in paraffin blocks, and sectioned at a thickness of 5 µm, followed by immunofluorescent staining.

The primary antibodies used were rabbit anti-β-catenin (Cell Signaling #9562) and mouse anti-AR (Santa Cruz, sc-7305). The fluorophore-conjugated secondary antibodies were goat anti-rabbit IgG conjugated with Alexa Fluor 488 (Abcam, ab150077) and goat anti-mouse IgG conjugated with Cyanine5 (Invitrogen, a10524).

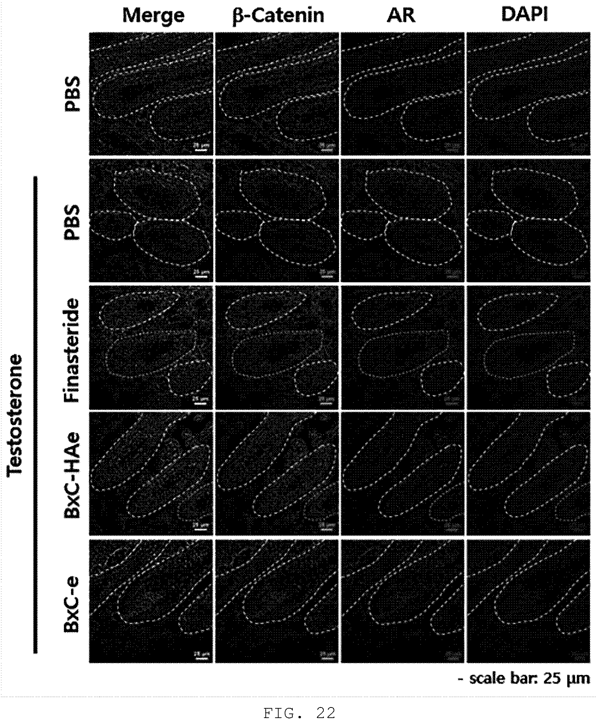

After completion of the antibody reactions, DAPI staining was performed, and fluorescence patterns were analyzed using a Nikon ECLIPSE Ti2 fluorescence microscope and NIS-Elements software. The results are shown in FIGS. 22-24, and in Tables 19 and 20.

**TABLE 19**

| | Testosterone -untreated | Testosterone-treated | | | |
|---|---|---|---|---|---|
| | | Contro l | 1^{st} Test group (Finasterid e treated) | 2^{nd} Test group (BxC-e treated ) | 3^{rd} Test group (BxC-HAe treated ) |
| Fluorescenc e intensity | 1 | 2.32 | 1.56 | 1.85 | 1.01 |

**TABLE 20**

| | Testosteron e-untreated | Testosterone-treated | | | |
|---|---|---|---|---|---|
| | | Contro l | 1^{st} Test group 1 (Finasterid e treated) | 2^{nd} Test group (BxC-etreated ) | 3^{rd} Test group (BxC-HAe treated ) |
| Fluorescenc e intensity | 1 | 0.57 | 1.05 | 0.60 | 1.09 |

According to the analysis: for AR fluorescence intensity, testosterone-treated mice exhibited a value of 2.32 relative to the untreated group; Test Substance 1 (finasteride) treatment reduced the AR fluorescence intensity to 1.56; and Test Substance 2 (BxC-e) treatment reduced the AR fluorescence intensity to 1.85.

For β-catenin fluorescence intensity, testosterone treatment resulted in a reduction to 0.57 relative to the untreated group. Test Substance 1 (Finasteride) treatment increased the fluorescence intensity to 1.05 while Test Substance 2 (BxC-e) treatment yielded a fluorescence intensity of 0.60.

With Test Substance 3 (BxC-HAe), the fluorescence intensity was 1.01 for AR and 1.09 for β-catenin compared to the testosterone-untreated group. These results indicate a statistically significant reduction in AR fluorescence and a significant recovery of β-catenin fluorescence compared to the control group. Therefore, it was confirmed that BxC-HAe is capable of treating or preventing androgenetic alopecia induced by testosterone.

### EXAMPLE 15: Evaluation of Effects of Conditioned Medium (CM) from Hyaluronic Acid-Pretreated iPSC-Derived Mesenchymal Stem Cells

### 15-1. Establishment of Conditioned Medium (CM) from BxC-HA Stem Cells

iPSC-derived mesenchymal stem cells (BxC-HA stem cells), prepared as described in Example 1, were cultured for 24 hours in high-glucose DMEM medium supplemented with 10% fetal bovine serum, 1% MEM Non-Essential Amino Acids Solution, and 40 µg/mL hyaluronic acid.

After completion of culturing, the BxC-HA stem cells were washed with D-PBS and further cultured for 72 hours in medium containing 15% exosome-depleted FBS.

Following the 72-hour incubation, the culture medium was collected and centrifuged at 300×g for 10 minutes to remove remaining cells and debris. The resulting conditioned medium (CM) was collected and used for the following experiment.

### 15-2. Evaluation of Recovery of Impaired Wound Healing in Human Dermal Papilla Cells

To determine whether the composition of the present disclosure promotes the recovery of reduced wound healing in human dermal papilla cells, the culture supernatant (CM) obtained in Example 15-1 was applied to the cells, and wound healing rate was evaluated after 24 hours.

Specifically, 25,000 human dermal papilla cells were seeded per well in a 12-well plate and incubated at 37 °C under 5% CO₂ for 24 hours in the basic medium described in Table 1. A cross-shaped wound was created using a 1mL pipette tip, and the cells were washed three times with 1× D-PBS to remove debris.

For experimental grouping: the testosterone-untreated group received 0.05% (v/v) DMSO in the basal medium; the control group was treated with 50 µM testosterone; the experimental group was treated with 50 µM testosterone and 50% (v/v) BxC-HA stem cell supernatant (CM) in combination. After treatment with each substance, all groups were incubated for an additional 24 hours after treatment.

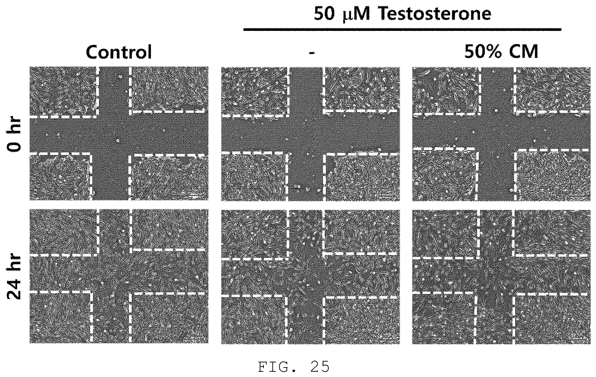

Cell layer recovery was observed at the time of treatment and 24 hours post-treatment using the Dixi eXcope imaging system, and images obtained in each medium were shown in FIG. 25.

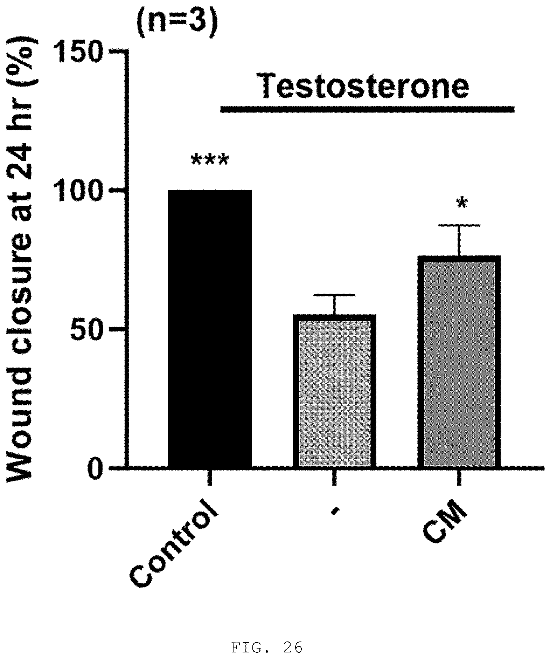

The images were quantitatively analyzed using ImageJ software. Three independent experiments were conducted per group, and the average values were calculated and presented in FIG. 26 and Table 21. Results were normalized relative to the mean value of the testosterone-untreated group, which was set to 100%.

**TABLE 21**

| | Testosterone -untreated | 50 µM Testosterone-treated | |
|---|---|---|---|
| | | Control | BxC-HA stem cell culture supernatant (CM) treated |
| Wound healing recovery after 24 hours (%) | 100 | 55.5 | 76.5 |

The analysis results indicate that treatment with testosterone reduced the wound healing rate by approximately 45% compared to the testosterone-untreated group while treatment with BxC-HA stem cell culture supernatant (CM) resulted in only a 24% reduction in wound healing rate compared to the testosterone-untreated group.

These results indicate that the CM from BxC-HA stem cells significantly improved wound healing compared to the control group, even under testosterone treatment, thereby confirming that BxC-HA stem cell culture supernatant (CM) markedly promotes recovery of human dermal papilla cells.

### 15-3. Promotion of Growth Factor Expression in Human Dermal Papilla Cells

To evaluate whether the composition of the present disclosure promotes the expression of growth factors known to be important for hair growth, namely IGF1 (Insulin-like Growth Factor 1) and EGF (Epidermal Growth Factor), human dermal papilla cells were treated with the culture supernatant (CM) obtained from BxC-HA stem cells as described in Example 15-1, and gene expression levels were assessed after 24 hours.

Specifically, 25,000 human dermal papilla cells were seeded in each well of a 12-well plate and cultured in basic medium at 37°C under 5% CO₂ for 24 hours. The composition of the basic medium was the same as described in Table 1.

The groups were prepared as follows: the testosterone-untreated group was treated with 0.05% (v/v) DMSO in the basic medium; the control group was treated with 50µM testosterone; and the experimental group was treated with a combination of 50µM testosterone and 50% (v/v) culture supernatant (CM) from BxC-HA stem cells. After treatment with respective substance, all samples were incubated for 24 hours after treatment.

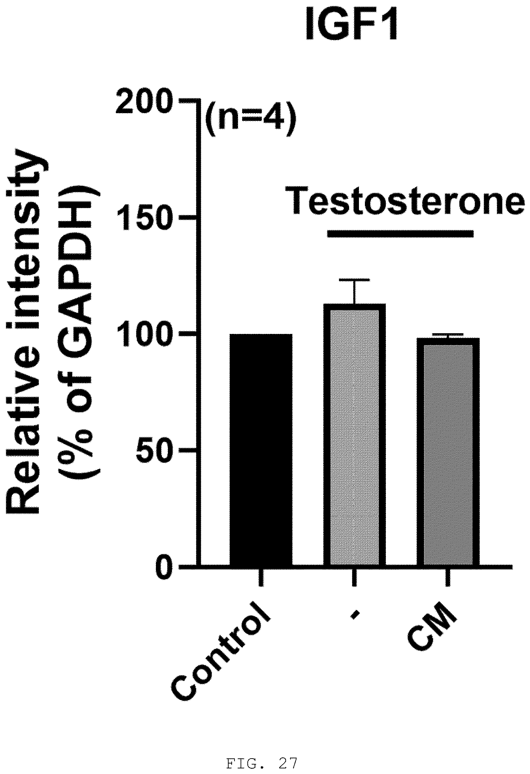

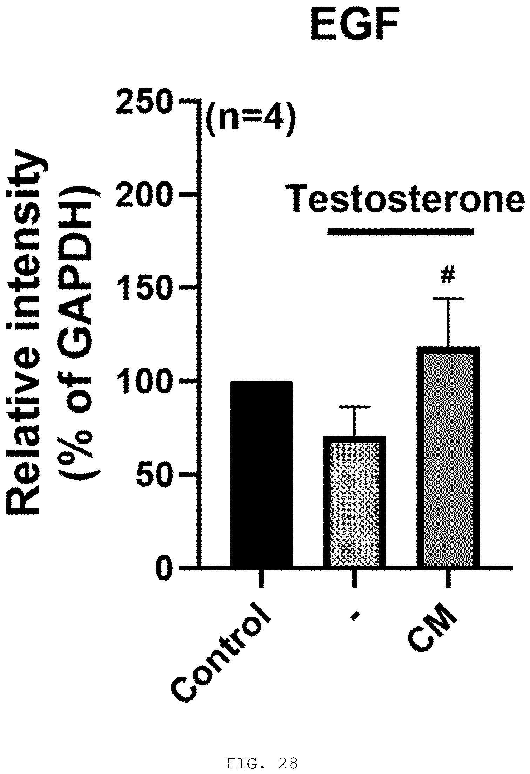

After completion of the incubation, cells were collected from each well, and total RNA was extracted using TRIzol^{™} (Invitrogen). The mRNA levels of IGF1 and EGF were quantified via real-time PCR (Applied Biosystems). Relative gene expression was normalized to GAPDH using the 2^{-ΔΔCt} method, and the results were presented relative to the untreated testosterone group, which was set at 100%. The results were shown in FIG. 27 and Table 22 for IGF1, and in FIG. 28 and Table 23 for EGF.

**TABLE 22**

| | Testosterone -untreated | 50 µM Testosterone-treated | |
|---|---|---|---|
| | | Control | BxC-HA stem cell culture supernatant (CM) treated |
| Relative expression of IGF1 gene (%) | 100 | 112.9 | 98.2 |

**TABLE 23**

| | Testosterone-untreated | 50 µM Testosterone-treated | |
|---|---|---|---|
| | | Control | BxC-HA stem cell culture supernatant (CM) treated |
| Relative expression of EGF gene (%) | 100 | 70.6 | 118.6 |

As a result of quantitative analysis, the expression level of the IGF1 gene was 112.9% of that in the untreated group when treated with testosterone in human mammary gland papilla cells, and 98.2% of that in the untreated group when treated with the culture supernatant (CM) of BxC-HA stem cells, which was the test substance. There was no statistically significant change.

On the other hand, the expression level of the EGF gene decreased to 70.6% compared to the untreated testosterone group when testosterone was treated in human hair follicle cells, and increased to 118.6% compared to the untreated testosterone group when treated with the culture supernatant (CM) of BxC-HA stem cells, which is a test substance. This indicates that the expression level of the EGF gene significantly increased at a statistically significant level compared to the control group. Through this analysis, it was confirmed that the culture supernatant (CM) of BxC-HA stem cells can promote hair growth by promoting EGF gene expression in human hair follicle cells, thereby exhibiting a treatment or prevention effect on hair loss.

### 15-4. Inhibition of Hair Follicle Growth-Inhibitory Factor Expression in Human Dermal Papilla Cells

To evaluate whether the composition of the present disclosure inhibits the expression of TGF-β1 (Transforming Growth Factor-beta 1) and IL-6 (Interleukin-6), which are known to shorten the anagen phase and induce catagen and telogen phases of the hair follicle cycle, human dermal papilla cells were treated with the cell supernatant (CM) obtained from BxC-HA stem cells described in Example 15-1. Gene expression levels of TGF-β1 and IL-6 were compared after 24 hours.

Specifically, 25,000 human dermal papilla cells were seeded into each well of a 12-well plate and incubated in basic medium at 37°C under 5% CO₂ for 24 hours. The composition of the basic medium was the same as described in Table 1.

The groups were set up as follows: the testosterone-untreated group was treated with 0.05% (v/v) DMSO in the basic medium; the control group was treated with 50 µM testosterone; and the experimental group was treated with 50µM testosterone and 50% (v/v) conditioned medium (CM) from BxC-HA stem cells. After treatment with respective substances, all groups were cultured for 24 hours after treatment.

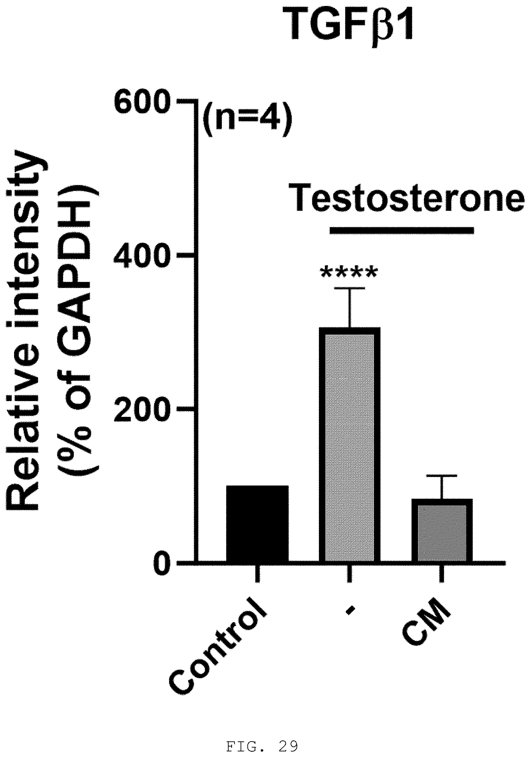

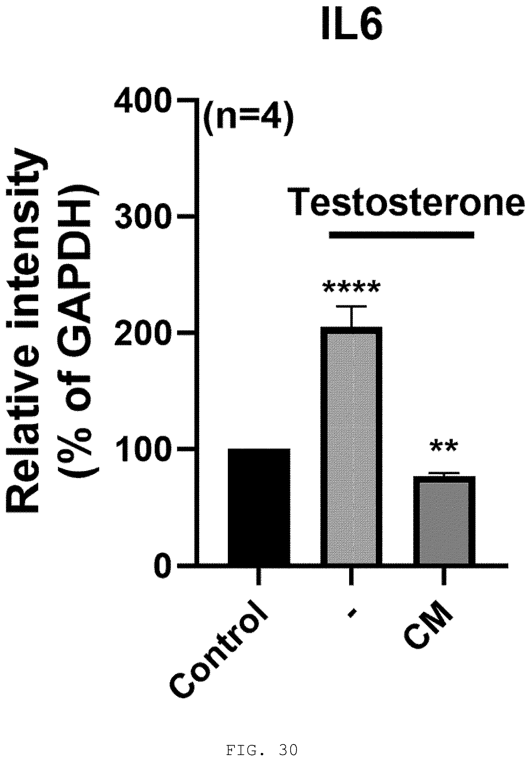

After completion of the incubation, cells were collected from each medium. Total RNA according to each medium was extracted using TRIzol^{™} (Invitrogen). Then, gene expression levels of TGF-β1 and IL-6 were measured from the equal amount of the total RNA extracted from each medium using Real-Time PCR (Applied Biosystems). Each expression value was normalized against GAPDH using the 2^{-ΔΔCt} method, and expression levels were expressed relative to the testosterone-untreated group set to 100%. The quantification results were presented in FIG. 29 and Table 24 for TGF-β1, and FIG. 30 and Table 25 for IL-6.

**TABLE 24**

| | Testosterone-untreated | 50 µM Testosterone-treated | |
|---|---|---|---|
| | | Control | BxC-HA stem cell culture supernatant (CM) treated |
| Relative expression of TGF-β1 gene (%) | 100 | 306.2 | 83.5 |

**TABLE 25**

| | Testosterone-untreated | 50 µM Testosterone-treated | |
|---|---|---|---|
| | | Control | BxC-HA stem cell culture supernatant (CM) treated |
| Relative expression of IL-6 gene (%) | 100 | 205.2 | 76.8 |

According to the quantification results, TGF-β1 gene expression increased to 306.2% in the testosterone-treated group compared to the testosterone-untreated group whereas the expression in the group treated with the CM from BxC-HA stem cells decreased significantly to 83.5% relative to the untreated group.

Likewise, IL-6 gene expression increased to 205.2% in the testosterone-treated group compared to the untreated group. Treatment with CM from BxC-HA stem cells significantly reduced IL-6 expression to 76.8% relative to the untreated group.

These results confirm that the culture supernatant (CM) from BxC-HA stem cells significantly suppresses the expression of TGF-β1 and IL-6-genes that inhibit hair follicle growth-under testosterone treatment, thereby supporting sustained hair follicle growth.

### Industrial Applicability

The present disclosure relates to a composition for preventing or treating hair loss, including exosomes derived from stem cells and a method for preparing same. More specifically, the present disclosure relates to a composition including exosomes, which are isolated from mesenchymal stem cells or a culture thereof and exhibit excellent promoting effects on growth and recovery of human dermal papilla cells, thereby being useful for preventing or treating hair loss.

## Claims

1. A pharmaceutical composition for treatment, prevention, alleviation, or suppression of hair loss, comprising exosomes isolated from mesenchymal stem cells (MSCs) derived from induced pluripotent stem cells (iPSCs) as an active ingredient.

2. The pharmaceutical composition of claim 1, wherein the induced pluripotent stem cell-derived mesenchymal stem cells are differentiated from progenitor cells of induced pluripotent stem cells-derived mesenchymal stem cells that do not express stage-specific embryonic antigen 4 (SSEA-4) protein.

3. The pharmaceutical composition of claim 1, wherein the induced pluripotent stem cells are human-derived induced pluripotent stem cells.

4. The pharmaceutical composition of claim 1, wherein the induced pluripotent stem cell-derived mesenchymal stem cells are pretreated with a pretreatment substance.

5. The pharmaceutical composition of claim 4, wherein the pretreatment substance is hyaluronic acid.

6. A cosmetic composition for preventing hair loss, strengthening hair roots, recovering hair follicles, or promoting hair growth, comprising exosomes isolated from induced pluripotent stem cell-derived mesenchymal stem cells (iPSCs) as an active ingredient.

7. The cosmetic composition of claim 6, wherein the induced pluripotent stem cell-derived mesenchymal stem cells are differentiated from progenitor cells of mesenchymal stem cells that do not express stage-specific embryonic antigen 4 (SSEA-4) protein.

8. The cosmetic composition of claim 6, wherein the induced pluripotent stem cells are human-derived induced pluripotent stem cells.

9. The cosmetic composition of claim 6, wherein the induced pluripotent stem cell-derived mesenchymal stem cells are pretreated with a pretreatment substance.

10. The cosmetic composition of claim 9, wherein the pretreatment substance is hyaluronic acid.

11. A method for preparing a pharmaceutical composition for treating hair loss, the method comprising:
a first culturing step of culturing induced pluripotent stem cells in a medium;
a selective culturing step of isolating SSEA-4(-) cells that do not express SSEA-4 protein from the cultured induced pluripotent stem cells and culturing same to differentiate into BxC stem cells;
a second culturing step of culturing the BxC stem cells to differentiate into mesenchymal stem cells;
a pretreatment step of pretreating the mesenchymal stem cells with hyaluronic acid;
a production step of culturing the pretreated mesenchymal stem cells to produce exosomes; and
a collection step of obtaining the culture supernatant from the mesenchymal stem cells or the culture thereof.

12. The method of claim 11, further comprising an isolation step of isolating exosomes from the culture supernatant.
